# EUROPEAN PATENT APPLICATION

(11) **EP 4 596 098 A1**
(43) Date of publication of application: **06.08.2025**
(21) Application number: 23873101.2
(22) Date of filing: 26.09.2023
(51) Int. Cl.: B01F 33/30, B01F 33/3031, B33Y 10/00, B33Y 80/00, B01F 101/22, B82Y 5/00

(54) **MICROFLUIDIC MIXING STRUCTURE AND MICROFLUIDIC MIXING APPARATUS COMPRISING SAME**

(30) Priority: 30.09.2022 KR 20220124937; 25.09.2023 KR 20230128550
(71) Applicant: POSTECH Research and Business Development Foundation, Pohang-si, Gyeongsangbuk-do 37673 (KR)
(72) Inventor: KANG, Byung Kwon, Daejeon 35337 (KR); NA, Gi Su, Pohang-si, Gyeongsangbuk-do 37673 (KR); KIM, Dong Pyo, Pohang-si, Gyeongsangbuk-do 37673 (KR); KIM, Kyungjin, Seoul 06170 (KR); YANG, Joosung, Ansan-si, Gyeonggi-do 15610 (KR); CHOI, Kang Hyun, Ansan-si, Gyeonggi-do 15610 (KR); KIM, Uk-Il, Ansan-si, Gyeonggi-do 15610 (KR); LEE, Joo Young, Ansan-si, Gyeonggi-do 15610 (KR)
(74) Representative: Jung, Minkyu
(86) International application number: PCT/KR2023/014811
(87) International publication number: WO 2024/071987

(57) **Abstract**

Disclosed are a microfluidic mixing structure and a microfluidic mixing apparatus comprising same. The microfluidic mixing structure according to an aspect of the present invention is for mixing a first fluid and a second fluid while moving them in a first direction and comprises: a first mixing unit including a first body which has an inlet through which the first fluid and the second fluid are introduced, and a first space in which the first fluid and the second fluid are mixed; and a second mixing unit which is provided on the rear side of the first mixing unit when viewed in the first direction, and includes a second body having a second space through which a mixed fluid obtained in the first mixing unit flows, wherein the first space in the first mixing unit and the second space in the second mixing unit are provided on opposite sides of an imaginary line extending in the first direction, the flow direction of the mixed fluid, in which the mixed fluid is introduced from an outlet of the first mixing unit into an inlet of the second mixing unit, is opposite to the first direction, and the first mixing unit and the second mixing unit include curved surfaces that are bilaterally symmetric with respect to the center in the width direction when viewed in the first direction.

## Description

### TECHNICAL FIELD

The present invention relates to a microfluidic mixing structure and a microfluidic mixing apparatus having the same.

### BACKGROUND

Nanotechnology has attracted great attention in the field of drug delivery due to its advantages such as specific cell targeting, enhanced permeation and retention (EPR) effects, and reduced cytotoxicity, and has overcome the limitations of existing delivery methods by overcoming biological barriers within organs. To maximize consistent therapeutic efficacy, precise control over size is important for specific diseases and target organs for effective treatment in conjunction with Good Manufacturing Practices (GMP) and clinical trials. To pursue these goals, the application of microfluidic technology has emerged as an essential approach that enables high material delivery and precise system control.

However, continuous nanoparticle production via microfluidics raises challenges affecting solution mixing, coagulation, and consistency of physicochemical properties. Particles that are outside the target size may accumulate in unwanted organs or cells, causing toxicity. In order to minimize side effects, it is very important in the design process of a microfluidic mixer to maximize mixing efficiency between solutions to achieve highly uniform nanoparticles. Conventional photolithography has limited application productivity due to solvent selectivity and flow issues caused by poor uniformity. Injection molding is a costly and less accessible alternative. 3D printing shows promise because it offers solvent stability and a variety of monolithic designs. However, lower resolution requires larger channels to be designed for precise and highly reproducible fabrication, which reduces mixing efficiency. Due to these limitations, operation at high flow rates is required to overcome mixing efficiency, which consumes excess reagents during particle optimization. Baffles inside the mixer are another alternative to overcome slow mixing limitations and improve mixing by inducing secondary flows. However, the widely used 2D baffle is prone to dead volume and nanoparticle adsorption, which reduces the performance of the mixer. Clogging poses additional challenges to scale-up, which is essential to addressing the fundamental problem of low productivity in microfluidics. Aggregated nanoparticles adsorbed inside the mixer or flow distributor cause pressure irregularities at each outlet, and the uneven flow distribution drastically reduces the production efficiency of nanoparticles with consistent characteristics within the scale-up device. Therefore, there is an urgent need for a new mixer that is capable of high-speed mixing while preventing clogging even during long-term operation and allowing convenient scale-up.

### DISCLOSURE

### TECHNICAL PROBLEM

The present invention aims to solve the above problems, and the present invention is directed to providing a microfluidic dual-vortex mixing structure that integrates micro Dean vortices to generate two symmetric counter-rotating enhanced vortices.

The present invention is also providing a dual vortex mixing structure (DVM) capable of forming two symmetrical vortices of very high intensity through 3D hemispherical baffles to enable ultra-high mixing within the mixer while continuously producing polymer and lipid-based nanoparticles.

The present invention is also providing a microfluidic mixing structure having a 3D baffle structure capable of ultra-high-speed mixing using a 3D printer, and providing an integrated/assembled microfluidic mixing apparatus for mass production of nanoparticles by connecting the microfluidic mixing structure and a flow distributor.

The problems of the present invention are not limited to those mentioned above, and other problems not mentioned will be clearly understood by those of ordinary skill in the art from the following description.

### TECHNICAL SOLUTION

According to an aspect of the present invention, provided is a microfluidic mixing structure for mixing a first fluid and a second fluid while moving them in a first direction, the microfluidic mixing structure including: a first mixing unit including a first body which has an inlet through which the first fluid and the second fluid are introduced, and a first space in which the first fluid and the second fluid are mixed; and a second mixing unit which is provided on the rear side of the first mixing unit when viewed in the first direction, and comprises a second body having a second space through which a mixed fluid obtained in the first mixing unit flows, wherein the first space in the first mixing unit and the second space in the second mixing unit are provided on opposite sides of an imaginary line extending in the first direction, wherein the flow direction of the mixed fluid, in which the mixed fluid is introduced from an outlet of the first mixing unit into an inlet of the second mixing unit, is opposite to the first direction, and the first mixing unit and the second mixing unit comprise curved surfaces that are bilaterally symmetric with respect to the center in the width direction when viewed in the first direction.

In this case, the first space in the first mixing unit and the second space in the second mixing unit may include one of an elliptical cross-section and a semicircular cross-section when viewed in the first direction.

In this case, the first space in the first mixing unit and the second space in the second mixing unit may be formed in a hemispherical shape.

In this case, the second mixing unit may be positioned above the first mixing unit in the up-down direction, and the inlet of the first mixing unit may be arranged to face upward.

In this case, the first space in the first mixing unit and the second space in the second mixing unit may be formed with the same area of each inlet and outlet.

In this case, the cross-sectional area of the central portion of the first space in the first mixing unit and the second space in the second mixing unit may be formed to be larger than the area of the inlet and outlet of each of the first space and the second space when viewed in the first direction.

In this case, the first space in the first mixing unit and the second space in the second mixing unit may have the inlet and outlet of each space formed as a closed curve shape in which a pair of circular arcs of the same diameter face each other and are in contact with each other when viewed in the first direction.

In this case, the microfluidic mixing structure may further include a third mixing unit including a third body placed on the rear side of the second mixing unit, when viewed in the first direction, and having a third space through which the mixed fluid passing through the second mixing unit flows.

In this case, the third mixing unit may have the same shape as at least one of the first mixing unit and the second mixing unit.

In this case, the third space in the third mixing unit may be arranged opposite to the second space in the second mixing unit with respect to an imaginary line extending in the first direction.

In this case, the microfluidic mixing structure may further include a first fluid inlet pipe through which the first fluid passes so that the first fluid is introduced into the inlet of the first mixing unit; and a second fluid inlet pipe through which the second fluid passes so that the second fluid is introduced into the inlet of the first mixing unit, and the first fluid inlet pipe and the second fluid inlet pipe may be arranged to be orthogonal, and one of the first fluid inlet pipe and the second fluid inlet pipe may be arranged to extend in the first direction.

In this case, the third space in the third mixing unit may have an outlet oriented upward, and a mixed fluid discharge pipe extending in the first direction may be connected to the outlet of the third space.

In this case, the first body, the second body, and the third body may be formed as a module integrated into one body.

In this case, a plurality of first mixing units, a plurality of second mixing units respectively connected to the plurality of first mixing units, and a plurality of third mixing units respectively connected to the plurality of second mixing units may be provided inside the module integrated into one body, a first fluid supply flow passage to which a first fluid supply pipe for supplying the first fluid into the interior of the one body is connected, and a second fluid supply flow passage to which a second fluid supply pipe for supplying the second fluid into the interior of the one body is connected may be provided in the one body, and the first fluid supply flow passage and the second fluid supply flow passage may be formed to be branched into a plurality of flow passages to supply the first fluid and the second fluid to the plurality of first mixing units.

In this case, the module integrated into one body may include 1, 2, 4, or 8 pairs of mixing flow passages therein, and each of the plurality of pair of mixing flow passages may be formed such that two first mixing units, two second mixing units, and two third mixing units are formed in a pair.

According to another aspect of the present invention, provided is a microfluidic mixing apparatus, including one or more microfluidic mixing structures described above; a first fluid feeder configured to supply the first fluid to each of the one or more microfluidic mixing structures; a second fluid feeder configured to supply the second fluid to each of the one or more microfluidic mixing structures.

In this case, the microfluidic mixing apparatus may further include a flow distributor configured to distribute the first fluid and the second fluid supplied from the first fluid feeder and the second fluid feeder to the one or more microfluidic mixing structures.

### ADVANTAGEOUS EFFECT

According to the above configuration, the 3D hemispherical baffle microfluidic mixing structure according to the present invention exhibits a rapid mixing efficiency of over 99% when passing through three hemispherical baffle structures under a wide range of flow rate conditions, and enables the production of uniform nanoparticles (size: ≤100 nm, PDI: ≤0.1).

In addition, the microfluidic mixing apparatus for mass production according to an exemplary embodiment of the present invention can be designed by mounting a uniform flow distribution device on an optimized mixing structure, and can be manufactured through two methods: an integrated type that integrates 4, 8, and 16 baffle mixers using a 3D printing technique, and an assembly type that uses an external flow distributor. The manufactured, mass-producible, modular, integrated microfluidic mixing apparatus equipped with a flow distributor and 16 mixing structures can provide low cost, on-demand structural deformability, ease of fabrication, and reproducibility, and can bridge the gap between academic research and industrial applications by addressing clinical and industrial productivity needs.

In addition, the microfluidic mixing structure according to an exemplary embodiment of the present invention exhibits rapid mixing characteristics even at a relatively low flow rate of several mL/min, and can produce highly uniform lipid, liposome, and polymer nanoparticles with a PDI value of below 0.1 and a size in the range of 50 to 100 nm. In particular, by evaluating lipid nanoparticles loaded with SARS-CoV-2 spike mRNA and validating protein expression in vitro and in vivo using Firefly luciferase (FLuc) mRNA, it is possible to achieve performance equivalent to that of a commercial toroidal mixer system. In addition, the powerful on-site dispersion of nanoparticles utilizing the force of three-dimensional extensional flow minimizes physical adhesion and aggregation, maintains consistent production performance without internal clogging during half-day operations, and facilitates drug quality control.

To enable efficient mixing even under low-flow conditions while maintaining long-term operation, the development of a user-friendly microfluidic mixer is required. To address these issues, the inventors of the present invention adopted a three-dimensional channel structure according to an exemplary embodiment of the present invention, which generates a highly robust vortex, thereby facilitating ultrafast mixing and achieving effective dispersion of nanoparticles. The microfluidic mixing structure and mixing apparatus according to an exemplary embodiment of the present invention demonstrate the capability to produce highly uniform nanoparticles of controllable size, as confirmed in in vitro and in vivo studies. In addition, the ability to prevent clogging during continuous operation, combined with optimization at the laboratory scale and the potential for parallel industrial production, positions the microfluidic mixing structure and mixing apparatus according to an exemplary embodiment of the present invention as a versatile and scalable tool that can significantly impact the advancement of nanoparticle-based therapeutics, diagnostics, and targeted drug delivery.

The optimized microfluidic mixing structure and mixing apparatus according to an exemplary embodiment of the present invention, designed through computational fluid dynamics (CFD) simulation, demonstrate performance equivalent to that of a commercially injection-molded toroidal mixer in terms of particle characteristics and protein expression in both in vitro and in vivo studies. In addition, by minimizing dead volume and dispersing the manufactured nanoparticles using a strong vortex effect, it prevents clogging more than 60 times better than a toroidal mixer and maintains consistent particle characteristics even during 12 hours of continuous operation. The microfluidic mixing structure and mixing apparatus according to an exemplary embodiment of the present invention, with a baffle length of only 3.9 mm, enable more efficient optimization by utilizing low-flow conditions and prevent clogging even during long-term operation, serving as a starting point for parallelized scale-up apparatuses for nanoparticle optimization at the laboratory scale and industrial production.

Advantageous effects of the present invention are not limited to the above-described effects, and should be understood to include all effects that can be inferred from the configuration of the invention described in the detailed description or claims of the present invention.

### BRIEF DESCRIPTION OF THE DRAWINGS

FIG. 1 is a conceptual diagram of a microfluidic mixing apparatus for mass production, which is equipped with a single microfluidic mixing structure and a uniform flow distributor for producing various nanoparticles (lipid nanoparticles, liposomes, and polymer nanoparticles) using a 3D printer, according to an exemplary embodiment of the present invention.
FIG. 2 is a perspective view illustrating a microfluidic mixing structure according to an exemplary embodiment of the present invention.
FIG. 3 is a side view illustrating a microfluidic mixing structure according to an exemplary embodiment of the present invention.
FIG. 4 is a plan view illustrating a microfluidic mixing structure according to an exemplary embodiment of the present invention.

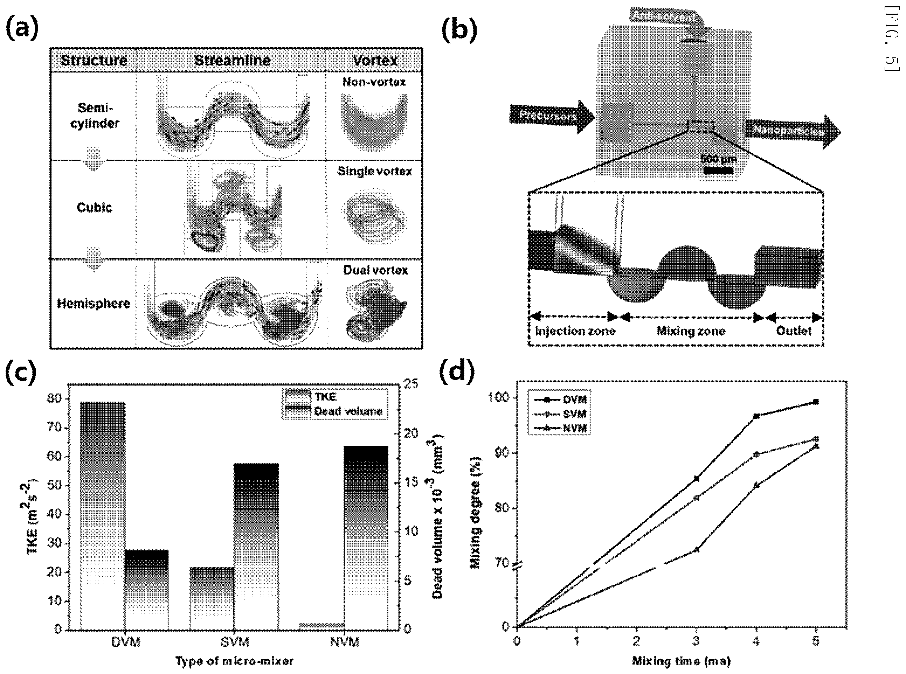

FIG. 5 is experimental data for design and CFD-based optimization of a microfluidic mixing structure according to an exemplary embodiment of the present invention, wherein FIG. 5(a) is a conceptual circuit diagram showing changes in vortex generation formed within a channel due to changes in a baffle structure, FIG. 5(b) is a complete circuit diagram and detailed components of the microfluidic mixing structure, FIG. 5(c) is a graph comparing flow intensity and dead volume size between a single vortex mixer (SVM) and a non-vortex-generating mixer (NVM) having the same specifications as the microfluidic mixing structure, and FIG. 5(d) is a graph showing the mixing efficiency of the microfluidic mixing structure (DVM) of the present invention, the single vortex mixer, and the non-vortex-generating mixer under a flow rate condition of 9 ml/min.
FIG. 6 shows the velocity profile, turbulent kinetic energy (TKE), and dead volume of the baffle microprocessor according to the structure of a single vortex mixer (SVM) and a non-vortex-generating mixer (NVM) that have the same specifications as a 3D hemispherical microfluidic mixing structure (dual vortex mixer) according to an exemplary embodiment of the present invention (volume of each baffle 0.19 mm³; baffle width 200 µm; ethanol 0.9 mL/min; water 8.1 mL/min, dark blue: dead volume).
FIG. 7 is a graph showing a comparison of mixing degree according to the mixing time of ethanol 0.9 mL/min and water 8.1 mL/min according to the structure of a single vortex mixer (SVM) and a non-vortex-generating mixer (NVM) having the same specifications as a 3D hemispherical microfluidic mixing structure (DVM: dual vortex mixer) according to an exemplary embodiment of the present invention.
FIG. 8 shows the calculated values of the mixing degree, turbulent kinetic energy, and dead volume at 5 ms for various baffle dimensions of a single vortex mixer (SVM) and a non-vortex-generating mixer (NVM) that have the same specifications as a 3D hemispherical microfluidic mixing structure (DVM: dual vortex mixer) according to an exemplary embodiment of the present invention (ethanol 0.9 mL/min, water 8.1 mL/min, dark blue: dead volume).
FIG. 9 is a diagram of a dual vortex generation according to the flow rate of ethanol and water when a flow rate ratio is 3 in a 3D hemispherical microfluidic mixing structure (DVM: dual vortex mixer) according to an exemplary embodiment of the present invention.
FIG. 10 is a diagram of a dual vortex generation according to the flow rate of ethanol and water when a flow rate ratio is 6 in a 3D hemispherical microfluidic mixing structure (DVM: dual vortex mixer) according to an exemplary embodiment of the present invention.
FIG. 11 is a diagram of a dual vortex generation according to the flow rate of ethanol and water when a flow rate ratio is 9 in a 3D hemispherical microfluidic mixing structure (DVM: dual vortex mixer) according to an exemplary embodiment of the present invention.
FIG. 12 is a photograph of a 3D hemispherical microfluidic mixing structure DVM (dual vortex mixer) (left) according to an exemplary embodiment of the present invention manufactured by digital light processing (DLP) 3D printing without tube connection and a 3D hemispherical microfluidic mixing structure (right) connected to a tube.
FIG. 13 shows a gas chromatography (GC) analysis result obtained to evaluate leaching after injecting ethanol and water into a 3D hemispherical microfluidic mixing structure according to an exemplary embodiment of the present invention for 12 hours (ethanol 0.9 mL/min; water 8.1 mL/min).
FIG. 14 is a graph showing the performance validation of a 3D hemispherical microfluidic mixing structure according to an exemplary embodiment of the present invention through the production of multipurpose nanoparticles. The normalized size distribution of (a) PCL nanoparticles, (b) PEG-PLGA nanoparticles, (c) POPC liposomes, and (d) mRNA-LNP was averaged from independent analysis (n=3), and the ratio shown in the graph corresponds to the ratio of the flow rate of the precursor solution to the flow rate of the poor solvent (in mL/min).
FIG. 15 shows the number Z-avg size and PDI values of (a) PCL, (b) PEG-PLGA nanoparticles, (c) POPC liposomes, and (d) Firefly luciferase (FLuc) mRNA encapsulated lipid nanoparticles (mRNA-LNP) in high concentrations; (e) the encapsulation efficiency of FLuc mRNA in (d); and (f) the performance index (Q) value defined as the reciprocal of the product of diameter (d) and polydispersity index (PDI).

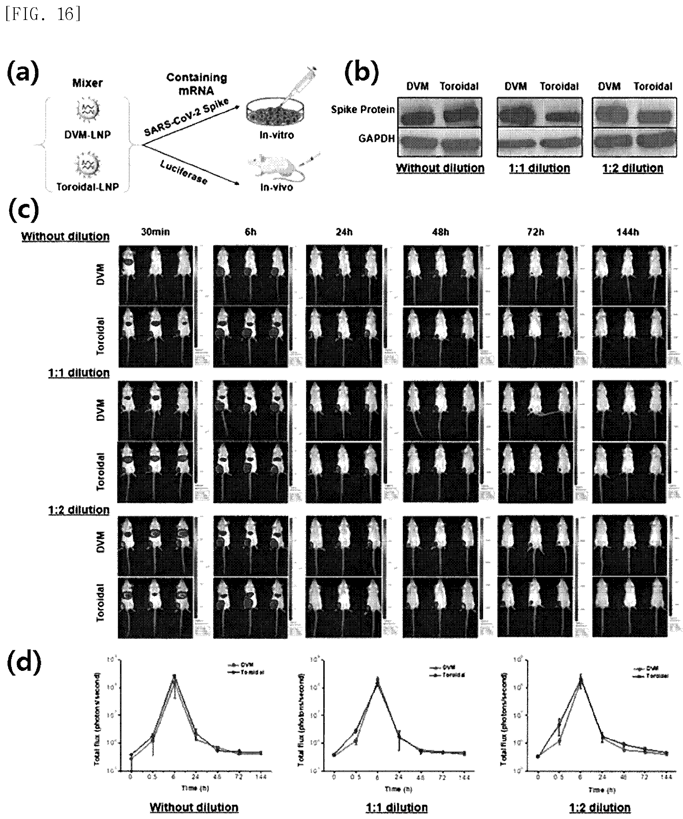

FIG. 16 is a comparison photograph of protein expression in vitro and in vivo of mRNA-LNPs generated by using different mixers. (a) mRNA-LNPs with identical formulation conditions were produced using a microfluidic mixing structure according to an exemplary embodiment of the present invention and a commercial toroidal mixer, and then directly purified or in-line diluted using PBS at a flow rate ratio of 1:1 or 1:2. mRNA-LNPs containing SARS-CoV-2 spike mRNA were utilized for in vitro applications, and mRNA-LNPs containing FLuc mRNA were used for in vivo biodistribution studies. (b) Western blot results after cell treatment with SARS-CoV-2 spike mRNA-LNPs diluted to the same concentration (spike protein: 190 kDa, GADPH: 37 kDa). (c) Bioluminescence imaging at specific time points following intravenous injection of FLuc mRNA-LNP into male BALB/c mice. (d) Quantification of total luminescence values for each formulation in groups of male BALB/c mice.
FIG. 17 is a schematic diagram of the assembly type of a 3D-printed microfluidic mixing structure.

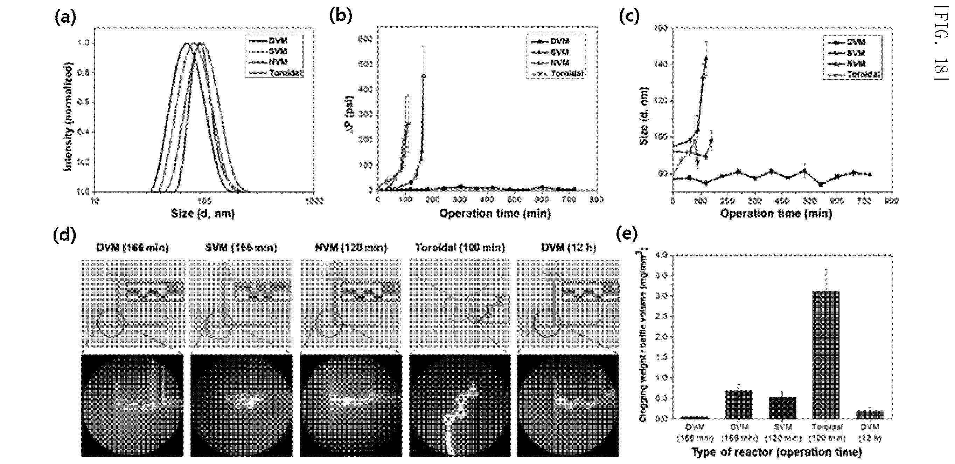

FIG. 18 is a graph of continuous production of TPE nanoparticles for long-term use validation. (a) Standardized size distribution of initial TPE nanoparticles with identical flow rate conditions generated by different microfluidic mixing structures. (b) Real-time analysis of the pressure difference inside the microfluidic mixing structure and (c) the average size of the nanoparticles. (d) Fluorescence visualization of clogged TPE nanoparticles under the same UV irradiation intensity within the decomposed structure. (e) Quantified ratio between the weight of clogged nanoparticles and the volume of a single structure.
FIG. 19 shows (a) normalized size distribution and (b) PDI values and numerical Z-avg sizes of TPE nanoparticles via DVM at various flow rate ratios (FRR = 3, 6, 9); and (c) normalized size distribution and (d) numerical Z-avg sizes and PDI values under fixed flow rate conditions of the microfluidic mixing structure (acetone 0.9 mL/min, DI water 8.1 mL/min).
FIG. 20 is a nanoparticle contact angle between a 3D-printed microfluidic mixing structure according to an exemplary embodiment of the present invention and a toroidal mixer.
FIG. 21 is a real working image for the continuous production of TPE nanoparticles using a 3D printed (a) microfluidic mixing structure according to an exemplary embodiment of the present invention and (b) a toroidal mixer.
FIG. 22 shows real-time observation values of TPE nanoparticles generated through (a) a microfluidic mixing structure (DVM) according to an exemplary embodiment of the present invention, (b) an SVM, (c) an NVM, and (d) a toroidal mixer.
FIG. 23 shows the weight of nanoparticles clogged inside the microfluidic mixing structure, quantified using UV-vis.

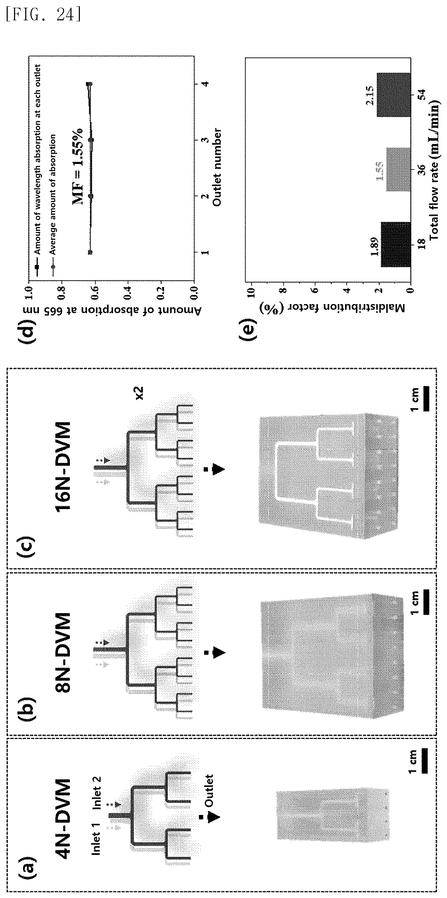

FIG. 24 is a conceptual diagram of an integrated mass production apparatus by parallelization of a flow distributor and a 3D microfluidic mixing structure for nanoparticle manufacturing. (a), (b), and (c) a conceptual diagram of a 3D printing integrated microfluidic mixing structure (4/8/16N-DVM) equipped with a uniform flow distributor and a photograph of the manufactured apparatus. (d), (e) After supplying rhodamine B staining solution and methylene blue staining solution, UV spectrum (665 nm) analysis of the solutions collected at each outlet, total flow rate of 36 mL/min., maldistribution factor (MF) according to the total flow rate of the 4N-DVM apparatus (ethanol flow rate: water flow rate = 1:9).
FIG. 25 is a block diagram in which 4 microfluidic mixing structures are integrally formed in parallel according to an exemplary embodiment of the present invention.
FIG. 26 is a conceptual diagram of an assembly-type mass production mixing apparatus of an external flow distributor and a plurality of 4N-DVMs. (a) a conceptual diagram of a 3-piece-assembled 8N-DVM, (b) a conceptual diagram of a 5-piece-assembled 16N-DVM.
FIG. 27 is a block diagram in which 8 microfluidic mixing structures are integrally formed in parallel according to an exemplary embodiment of the present invention.
FIG. 28 is a block diagram in which 16 microfluidic mixing structures are integrally formed in parallel according to an exemplary embodiment of the present invention.

### MODES OF THE INVENTION

Hereinafter, exemplary embodiments of the present invention will be described in detail so that those of ordinary skill in the art can readily implement the present invention with reference to the accompanying drawings. The present invention may be embodied in many different forms and is not limited to the embodiments set forth herein. In the drawings, parts unrelated to the description are omitted for clarity of description of the present invention, and throughout the specification, same or similar reference numerals denote same elements.

Terms and words used in the present specification and claims should not be construed as limited to their usual or dictionary definition. They should be interpreted as meaning and concepts consistent with the technical idea of the present invention, based on the principle that inventors may appropriately define the terms and concepts to describe their own invention in the best way.

Accordingly, the embodiments described in the present specification and the configurations shown in the drawings correspond to preferred embodiments of the present invention, and do not represent all the technical idea of the present invention, so the configurations may have various examples of equivalent and modification that can replace them at the time of filing the present invention.

It should be understood that the terms "comprise or include" or "have" or the like when used in this specification, are intended to describe the presence of stated features, numbers, steps, operations, elements, components and/or a combination thereof but not preclude the possibility of the presence or addition of one or more other features, numbers, steps, operations, elements, components, or a combination thereof.

The presence of an element in/on "front" , "rear" , "upper or above or top" or "lower or below or bottom" of another element includes not only being disposed in/on "front" , "rear" , "upper or above or top" or "lower or below or bottom" directly in contact with other elements, but also cases in which another element being disposed in the middle, unless otherwise specified. In addition, unless otherwise specified, that an element is "connected" to another element includes not only direct connection to each other but also indirect connection to each other.

Hereinafter, a microfluidic mixing structure and mixing apparatus according to an exemplary embodiment of the present invention will be described with reference to the drawings.

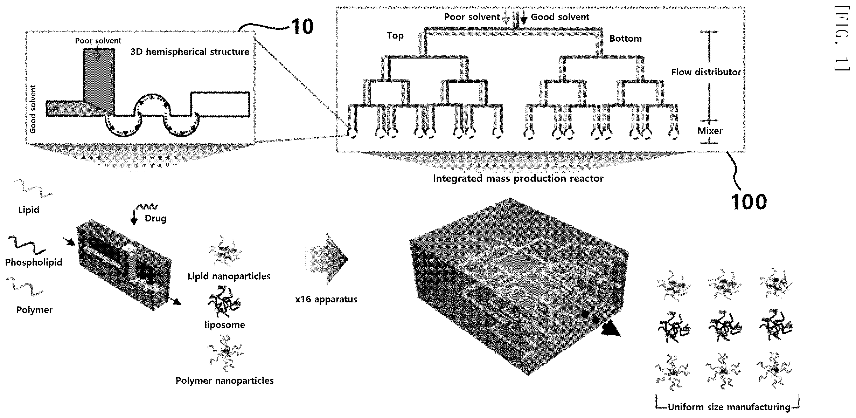

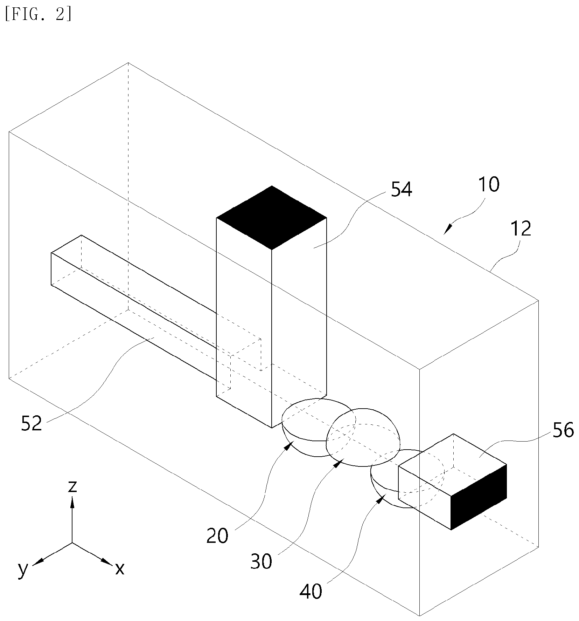

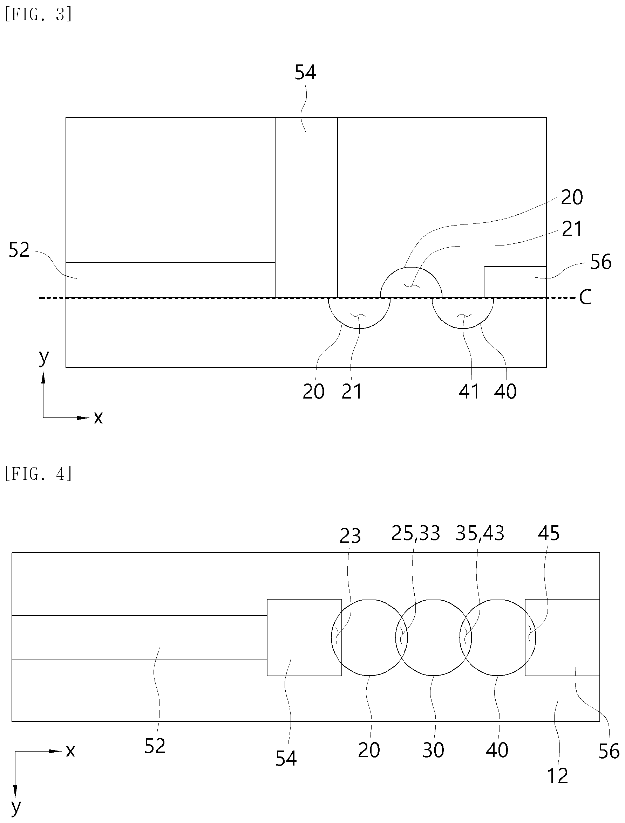

FIG. 1 is a conceptual diagram of a microfluidic mixing apparatus for mass production, which is equipped with a single microfluidic mixing structure and a uniform flow distributor for producing various nanoparticles (lipid nanoparticles, polyplexes, and polymer nanoparticles) using a 3D printer, according to an exemplary embodiment of the present invention. FIG. 2 is a perspective view illustrating a microfluidic mixing structure according to an exemplary embodiment of the present invention. FIG. 3 is a side view illustrating a microfluidic mixing structure according to an exemplary embodiment of the present invention. FIG. 4 is a plan view illustrating a microfluidic mixing structure according to an exemplary embodiment of the present invention.

FIG. 1 is a conceptual diagram of a microfluidic mixing structure 10 and a mixing apparatus 100 for single and mass production to produce uniform-sized drug delivery nanoparticles. The microfluidic mixing structure 10 may be manufactured using a 3D printer using a photocurable resin and has a hemispherical baffle structure. A hemispherical baffle structure, several hundred micrometers (900 µm) in size, is arranged in an alternating top-and-bottom pattern, inducing low-pressure and rapid vortex-flow mixing. This rapid mixing facilitates the manufacture of various nanoparticles in a uniform and small size, and the reproducibility of particle manufacture is also excellent.

Referring to FIGS. 2 to 4, a microfluidic mixing structure 10 according to an exemplary embodiment of the present invention may include a first mixing unit 20, a second mixing unit 30, and a third mixing unit 40.

The first mixing unit 20 may include a first body having an inlet 23 through which a first fluid and a second fluid are introduced, and having a first space 21 in which the first fluid and the second fluid are mixed. In this case, for example, the first fluid may be a good solvent, and the second fluid may be a poor solvent.

In addition, the second mixing unit 30 may include a second body with a second space 31 through which the mixed fluid mixed in the first mixing unit 20 flows by being placed on the rear side of the first mixing unit 20, when viewed in the first direction, that is, in the x-axis direction in FIG. 2. As can be seen from FIG. 2, in an embodiment of the present invention, the first body and the second body are formed as a single body 12. In this case, as another example, after the first body and the second body are formed as separate structures, the first space and the second space may be combined to allow fluid communication.

In this case, the first space 21 of the first mixing unit 20 and the second space 31 of the second mixing unit 30 may be arranged opposite to each other based on the virtual center line (C in FIG. 3) extending in the first direction; the flow direction of the mixed fluid flowing from the outlet 25 of the first mixing unit 20 to the inlet 33 of the second mixing unit 30 may be a direction intersecting the first direction, for example, a perpendicular direction; and the first mixing unit 20 and the second mixing unit 30 may include a curved surface that is bilaterally symmetrical with respect to a center in a width direction when viewed in the first direction.

In addition, the third mixing unit 40 may include a third body placed on the rear side of the second mixing unit 30, when viewed in the first direction, and having a third space 41 through which the mixed fluid passing through the second mixing unit 30 flows. In an embodiment of the present invention, the first to third bodies are formed of a single body 12.

In this case, the third space 41 of the third mixing unit 40 may be disposed opposite to the second space 31 of the second mixing unit 30 based on an imaginary line extending in the first direction.

In an embodiment of the present invention, the first space 21 of the first mixing unit 20 and the second space 31 of the second mixing unit 30 may include any one of an elliptical cross-section and a semicircular cross-section when viewed in the first direction.

In the present embodiment, the first space 21 of the first mixing unit 20, the second space 31 of the second mixing unit 30, and the third space 41 of the third mixing unit 40 may be formed in a hemispherical shape.

In the present embodiment, the second mixing unit 30 is located above the first mixing unit 20 and the third mixing unit 40 in the up-down direction, and the inlet 23 of the first mixing unit 20 is disposed to face the upward direction.

In this case, the inlet 25 of the first space 21 of the first mixing unit 20, the inlet 33 and the outlet 35 of the second space 31 of the second mixing unit 30, and the inlet 43 of the third space 41 are formed to have the same area.

In addition, when viewed in the first direction, the cross-sectional areas of the centers of the first space 21 of the first mixing unit 20, the second space 31 of the second mixing unit 30, and the third space 41 of the third mixing unit 40 are larger than the areas of the inlets 23, 33, 43 and outlets 25, 35, 45, respectively, of the first space 21, the second space 31, and the third space 41.

Referring to FIGS. 2 to 4, when viewed in the first direction, the outlet 25 of the first space 21 of the first mixing unit 20 and the inlet 33 of the second space 31 of the second mixing unit 30, the outlet 35 of the second space 31 of the second mixing unit 30 and the inlet 43 of the third space 41 of the third mixing unit 40 may have a closed curve shape in which a pair of circular arcs having the same diameter face each other and are in contact with each other.

Meanwhile, in an embodiment of the present invention, the microfluidic mixing structure 10 includes a first fluid inlet pipe 52 and a second fluid inlet pipe 54. The first fluid inlet pipe 52 is formed to allow the first fluid to pass therethrough so that the first fluid is introduced into the inlet of the first mixing unit 20, and the second fluid inlet pipe 54 is formed to allow the second fluid to pass therethrough so that the second fluid is introduced into the inlet of the first mixing unit 20.

In an embodiment of the present invention, the first fluid inlet pipe 52 and the second fluid inlet pipe 54 are disposed to be orthogonal, and one of the first fluid inlet pipe 52 and the second fluid inlet pipe 54 is disposed to extend in the first direction. In the present embodiment, the first fluid inlet pipe 52 extends in the first direction, that is, in the x-axis direction.

In addition, the third space 41 of the third mixing unit 40 has an outlet 45 oriented upward, and a mixed fluid discharge pipe 56 extending in the first direction is connected to the outlet 45 of the third space 41.

In an embodiment of the present invention, the first body, the second body, and the third body may be formed as a module integrated into one body 12. In the present embodiment, one integrated module-shaped body 12 may be formed by a 3D printer. However, the first to third bodies may be formed as separate bodies and then combined.

The inventors of the present invention focused on three aspects in developing a new micro mixing structure capable of maintaining ultra-high-speed mixing performance for a long period of time. First, the internal baffle structure within the channel forms a secondary flow, which improves mixing efficiency under consistent flow conditions. Second, the use of a circular channel with lower dead volume compared to a square channel improves mixing efficiency and facilitates continuous operation. Finally, the vortex generated inside the reactor further enhances the mixing efficiency and has a beneficial effect on the dispersion of nanoparticles, preventing adhesion and agglomeration.

The inventors of the present invention hypothesized that by using a circular channel and creating a larger number of secondary flows within it, more active vortices could be created, thereby maximizing mixing efficiency and enabling long-term operation. The amount of secondary flow formed may be controlled by adjusting the flow rate and intensity in the secondary flow direction along the axis of the primary flow.

FIG. 5 is experimental data for design and CFD-based optimization of a microfluidic mixing structure according to an exemplary embodiment of the present invention, wherein FIG. 5(a) is a conceptual circuit diagram showing changes in vortex generation formed within a channel due to changes in a baffle structure, FIG. 5(b) is a complete circuit diagram and detailed components of the microfluidic mixing structure, FIG. 5(c) is a graph comparing flow intensity and dead volume size between a single vortex mixer (SVM) and a non-vortex-generating mixer (NVM) having the same specifications as the microfluidic mixing structure, and FIG. 5(d) is a graph showing the mixing efficiency of the microfluidic mixing structure (DVM) of the present invention, the single vortex mixer, and the non-vortex-generating mixer under a flow rate condition of 9 ml/min.

Referring to FIGS. 5(a) to 5(d), in the case of a 2D baffle with a consistent channel height to introduce an additional flow direction, the formation of a single vortex may be determined by a subtle change in flow intensity. Semi-cylindrical baffles did not induce a clear change in fluid flow direction, thus inhibiting vortex formation. In contrast, cube baffles induce an additional flow perpendicular to the dominant flow axis due to their angular structure, resulting in a single vortex. However, when introducing a 3D hemispherical baffle structure, subsequent emissions of cohesive flows through narrow channels require a transverse path shape that is symmetrical to the diagonal side with respect to the dominant flow direction but with distinct micro-curvature radii. As a result, each flow element produces dean vortices at various intensities along the axial axis. The integration of components is driven by a back-pressure gradient, experiencing fast flow along the wall boundary due to centrifugal forces within the opposite shape of the structure. The resulting distribution appears as a visible secondary flow, characterized by a diagonally oriented bisymmetric recirculation vortex with respect to the primary flow.

### Modelling and CFD Study of 3D Hemispherical Systems

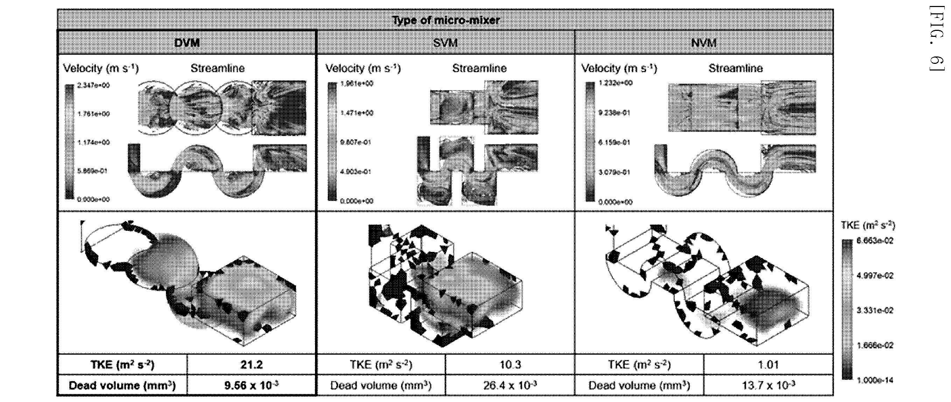

FIG. 6 shows the velocity profile, turbulent kinetic energy (TKE), and dead volume of the baffle microprocessor according to the structure of a single vortex mixer (SVM) and a non-vortex-generating mixer (NVM) that have the same specifications as a 3D hemispherical microfluidic mixing structure (dual vortex mixer: DVM) according to an exemplary embodiment of the present invention (volume of each baffle 0.19 mm³; baffle width 200 µm; ethanol 0.9 mL/min; water 8.1 mL/min, dark blue: dead volume).

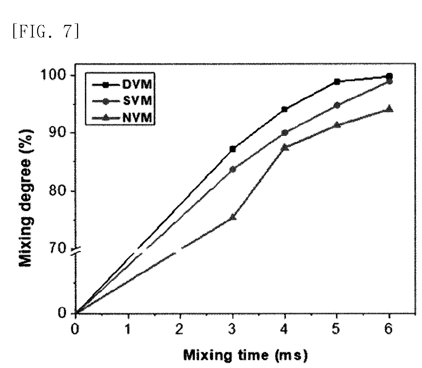

FIG. 7 is a graph showing a comparison of mixing degree according to the mixing time of ethanol 0.9 mL/min and water 8.1 mL/min according to the structure of a single vortex mixer (SVM) and a non-vortex-generating mixer (NVM) having the same specifications as a 3D hemispherical microfluidic mixing structure (DVM: dual vortex mixer) according to an exemplary embodiment of the present invention.

As can be seen in FIGS. 6 and 7, the top three virtual channel models, each including an injection region and an outlet, were designed with the same volume and structure width to perform a comprehensive numerical comparison of various structures based on computational fluid dynamics (CFD) simulations. These models include a non-vortex mixer (NVM) with semi-cylindrical baffles, a single vortex mixer (SVM) with cubic baffles, and a dual vortex mixer (DVM) with hemispherical baffles. Ethanol and water were selected for simulation of lipid nanoparticle synthesis. The inventors of the present invention evaluated these micro-mixers based on turbulent kinetic energy (TKE), which represents vortex intensity, formation of dead volume within the baffle, and mixing efficiency over time. Due to the enhanced secondary flow, DVM outperformed SVM and NVM turbulent kinetic energies and exhibited the best mixing performance with minimal dead volume.

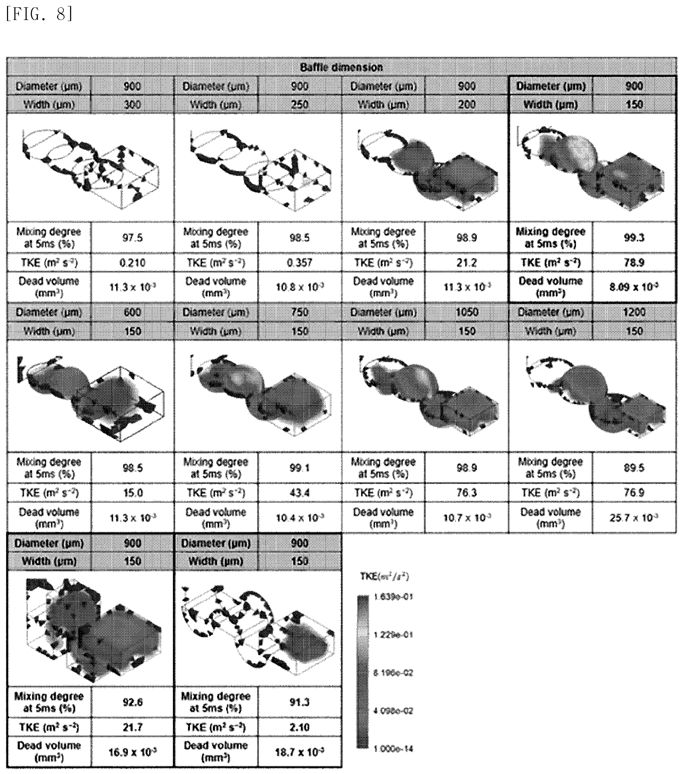

FIG. 8 shows the calculated values of the mixing degree, turbulent kinetic energy, and dead volume at 5 ms for various baffle dimensions of a single vortex mixer (SVM) and a non-vortex-generating mixer (NVM) that have the same specifications as a 3D hemispherical microfluidic mixing structure (DVM: dual vortex mixer) according to an exemplary embodiment of the present invention (ethanol 0.9 mL/min, water 8.1 mL/min, dark blue: dead volume).

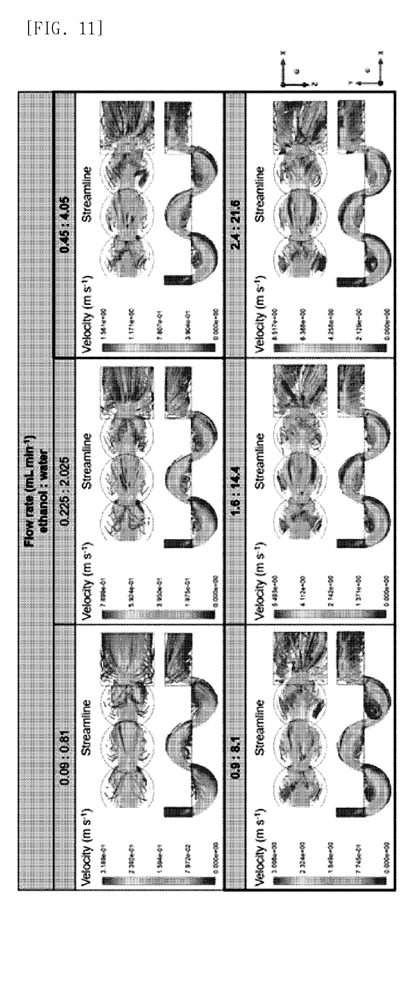

Referring to FIG. 8, further optimization of the dual vortex mixer included evaluating TKE, dead volume, and degree of mixing using various baffle dimensions at the same flow rate. The inventors of the present invention have found that reducing the baffle width improves mixing due to the effects of rapid reduction and expansion. The structural diameter affects not only the effect of the baffle itself, but also the adjustment of the internal volume. As the diameter increases, the residence time of a single baffle structure increases dramatically under the same flow conditions. This leads to a reduction in the number of baffle structures passing through the channel within the same residence time, making it difficult to assess the fundamental influence between the baffles. On the other hand, in the relatively small diameter region, the number of passing baffle structures increases, but the relative amplification effect decreases during the transition from one baffle to the next. Additionally, the slope of the dean vortex within the structure decreases, resulting in a decrease in the intensity of the vortex with respect to the secondary flow. As a result, as can be seen in FIG. 11, a diameter of 900 µm was finally selected to maximize the combination of the residence time for the entire mixer volume and the effects of each structure. Preferably, the spacing between baffles may be 50 to 300 µm, the baffle diameter may be 600 to 1200 µm, and the ratio of the spacing between baffles to the baffle diameter may be about 2 to 24.

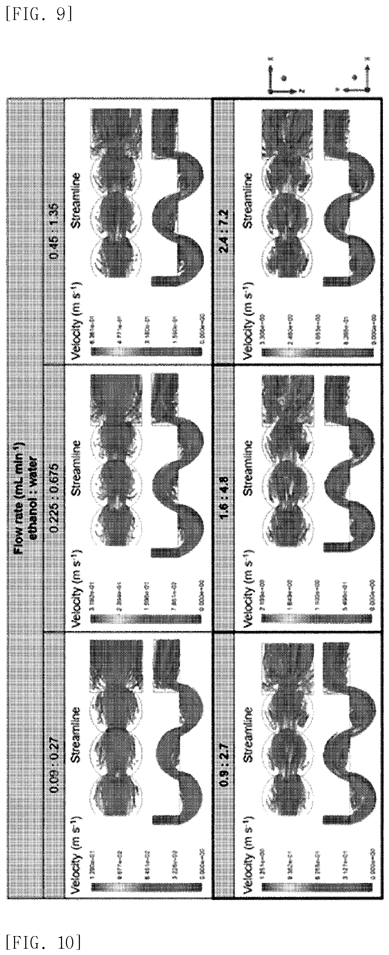

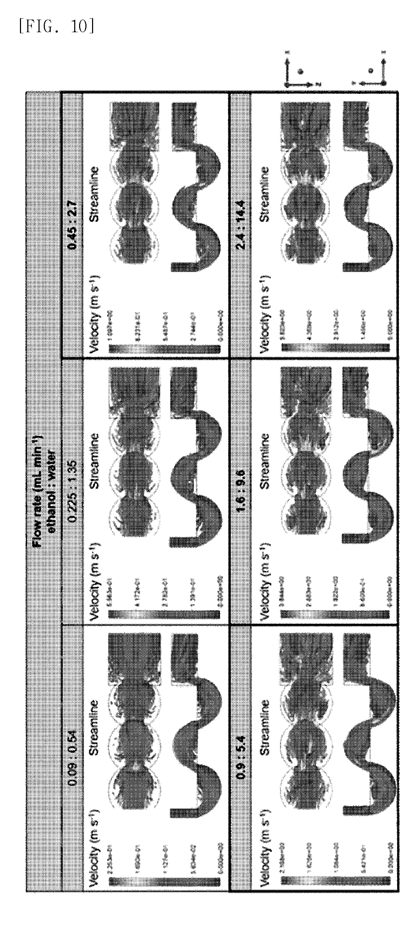

FIGS. 9 to 11 are diagrams of a dual vortex generation according to the flow rate of ethanol and water when a flow rate ratio is 3, 6, and 9, respectively, in a 3D hemispherical microfluidic mixing structure (DVM: dual vortex mixer) according to an exemplary embodiment of the present invention.

Referring to FIGS. 9 to 11, the optimized DVM achieves a TKE of 78.9 m² s⁻², which exceeds SVM by a factor of more than 3.5 and NVM by a factor of more than 38, and also has a dead volume of 8.09 x 10⁻³mm³. This was more than twice smaller than other models (see (d) in FIG. 5). The flow rates forming the symmetrical dual vortex were set to different flow rate ratios (FRR = 3, 6, 9). As can be seen in FIGS. 9 to 11, at FRR 3, ethanol flow ≥0.9 ml/min exhibited distinct dual vortices that were enhanced at higher FRR and used for actual nanoparticle production. Preferably, the flow rate ratio may be in the range of 1:1 to 1:9. In this case, the flow rate may be 0.9 mL/min or more for the first fluid and 2.7 mL/min or more for the second fluid.

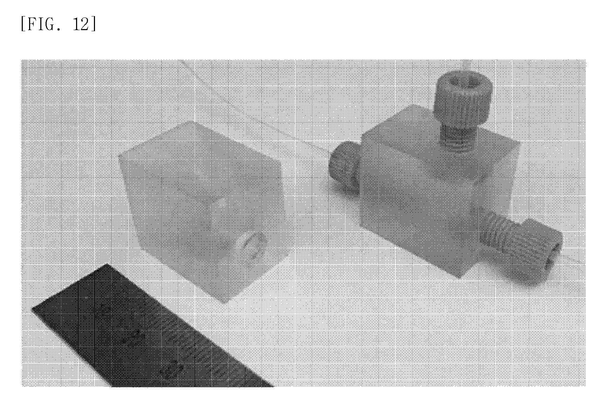

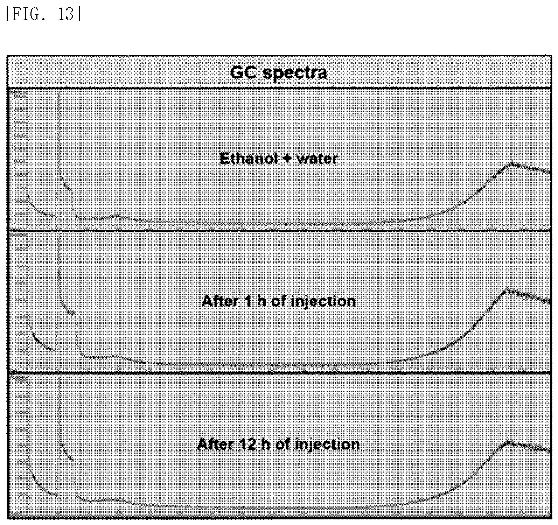

FIG. 12 is a photograph of a 3D hemispherical microfluidic mixing structure, that is, DVM (dual vortex mixer) (left) according to an exemplary embodiment of the present invention manufactured by digital light processing (DLP) 3D printing without tube connection and a 3D hemispherical microfluidic mixing structure (right) connected to a tube. FIG. 13 shows a gas chromatography (GC) analysis result obtained to evaluate leaching after injecting ethanol and water into a 3D hemispherical microfluidic mixing structure according to an exemplary embodiment of the present invention for 12 hours (ethanol 0.9 mL/min; water 8.1 mL/min).

As can be seen in FIG. 12, the final selected DVM may be manufactured using 3D printing resin. The chemical stability of the DVM fabricated in this manner was further validated through continuous alcohol-based solvent injection for 12 hours, as can be seen in FIG. 16, and confirmed through GC analysis.

### Fabrication of 3D hemispherical structures

3D printing not only allows for the easy fabrication of three-dimensional structures, but also allows for selective utilization of resins to achieve high chemical stability in organic solvents used in nanoparticle fabrication after curing. Among various printing methods, digital light processing (DLP) printing is widely known for achieving high resolution and is widely used to fabricate microfluidic channels with a size of less than 1 mm. The inventors of the present invention selected a poly(methyl methacrylate) (PMMA)-based resin that provides high stability for long-term operation and prevents device deformation under high pressure conditions when used with ethanol, which is commonly used in the production of lipid-based nanoparticles. As can be seen in FIG. 12, the entire structure of the DVM apparatus was rapidly fabricated (45 minutes per apparatus) using selected resins, including a 10-mm fluid stabilization section between the hemispherical baffle structure and the inlet and a 5-mm inlet and outlet section for fitting. It can be manufactured with very accurate dimensions through DLP printing.

In addition, referring to FIG. 13, the DVM apparatus manufactured using this resin was confirmed to have no leaching even after 12 hours of long-term ethanol injection using gas chromatography (GC) used to generate lipid-based nanoparticles.

Therefore, it can be confirmed that the microfluidic mixing structure and mixing apparatus according to an exemplary embodiment of the present invention have mechanical and chemical characteristics necessary for simple sample preparation and long-term continuous production of optimized particles in the process of optimizing nanoparticle characteristics.

### Production of nanoparticles for drug delivery

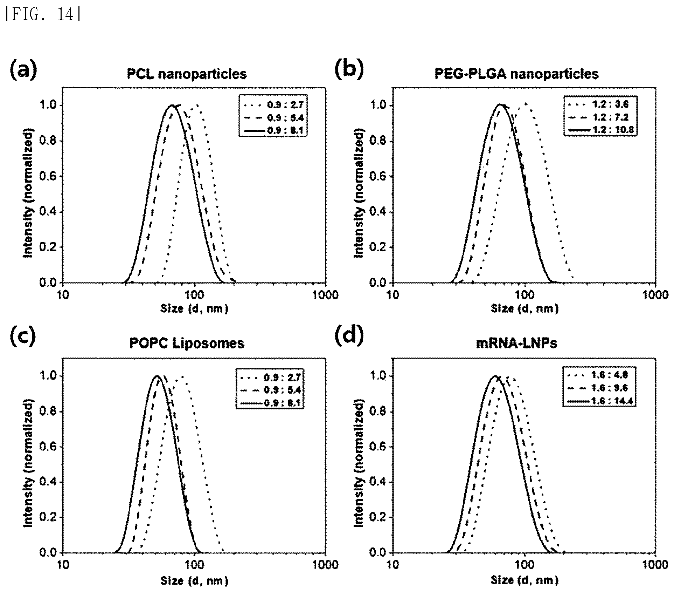

FIG. 14 is a graph showing the performance validation of a 3D hemispherical microfluidic mixing structure according to an exemplary embodiment of the present invention through the production of multipurpose nanoparticles. The normalized size distribution of (a) PCL nanoparticles, (b) PEG-PLGA nanoparticles, (c) POPC liposomes, and (d) mRNA-LNP was averaged from independent analysis (n=3), and the ratio shown in the graph corresponds to the ratio of the flow rate of the precursor solution to the flow rate of the poor solvent (in mL/min).

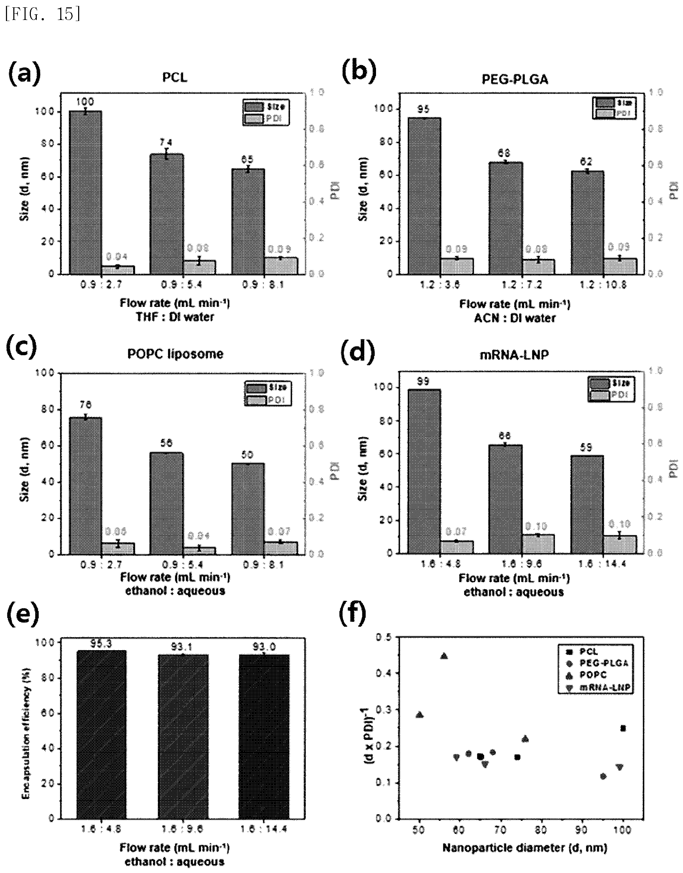

FIG. 15 shows the number Z-avg size and PDI values of (a) PCL, (b) PEG-PLGA nanoparticles, (c) POPC liposomes, and (d) Firefly luciferase (FLuc) mRNA encapsulated lipid nanoparticles (mRNA-LNP) in high concentrations; (e) the encapsulation efficiency of FLuc mRNA in (d); and (f) the performance index (Q) value defined as the reciprocal of the product of diameter (d) and polydispersity index (PDI).

DVM is a multipurpose platform for producing lipid- and polymer-based drug delivery nanoparticles with high uniformity and controllable size. To demonstrate the functionality of this platform, referring to FIG. 14, the inventors of the present invention selected polycaprolactone (PCL), poly(ethylene glycol)-b-poly(lactide-co-glycolide)(PEG-PLGA) nanoparticles, and 1-palmitoyl-2-oleoyl-sn-glycero-. 3-phosphocholine (POPC) liposomes and mRNA-encapsulating lipid nanoparticles (mRNA-LNPs) are FDA-approved representative substances. Because of the inefficient overall control of high-content polymers, diffusion mixing performed in a low flow rate range may result in the creation of large micron-sized aggregates. To demonstrate a wide range of concentration condition control, PCL was set to a concentration of 1 mg/ml using a conventional microfluidic approach, while PEG-PLGA was set to synthesize nanoparticles at a high concentration of 50 mg/ml, which would otherwise be unmanageable. Using dynamic light scattering (DLS) analysis, highly reproducible nanoparticles with diameters ranging from 50 nm to 100 nm and polydispersity indices (PDIs) up to 0.10 were produced through rapid mixing (see FIG. 14 (a), (b)).

These nanoparticles fall within the optimal range of stable and physiologically relevant nanoparticles and generally conform well to international nanoparticle specifications. In particular, the relatively large-capacity DVM with submillimeter diameter enables ultrafast mixing within the channel, indicating that high concentrations of precursors can be systematically controlled within microfluidic mixers.

The performance of the DVM apparatus outperforms that of other microfluidic apparatuses, especially when considering their excellent figure of merit (Q) values. The Q value, which represents the reciprocal of the product of liposome diameter (d) and polydispersity, i.e., Q = d⁻¹ PDI⁻¹, consistently exceeds 0.15 for each nanoparticle generated via DVM. In particular, for POPC liposomes, exceptionally high Q values in the range of 0.22 to 0.45 were calculated (see FIG. 14 (c) and FIG. 15 (c) and (f)). This value exceeds the previously reported value of 0.4, indicating the superior performance and superiority of DVM in terms of nanoparticle size and uniformity. By utilizing low FRR frequently used in nanoparticle manufacturing, the DVM platform according to an exemplary embodiment of the present invention can reduce the concentration process of liposome solutions for clinical use and reduce drug release from liposomes during the purification step. It should be noted that the internal volume of the DVM is relatively large compared to reported micromixers, which may potentially result in insufficient interaction between drug and carrier. To address this issue, the inventors of the present invention utilized the ability of LNPs to encapsulate firefly luciferase mRNA (FLuc-mRNA) and demonstrated that nanoparticles generated from DVM can carry large amounts of nucleic acids. 93% or more of the FLuc-mRNA was successfully encapsulated in each sample, indicating that sufficient electrostatic attraction was generated between the ionized lipid and mRNA, even though the microfluidic mixer was scaled up for large-volume production (see FIGS. 14(d), 15(d), and 15(e)).

### Protein expression of mRNA-LNPs in vitro/in vivo

FIG. 16 is a comparison photograph of protein expression in vitro and in vivo of mRNA-LNPs generated by using different mixers. (a) mRNA-LNPs with identical formulation conditions were produced using a microfluidic mixing structure according to an exemplary embodiment of the present invention and a commercial toroidal mixer, and then directly purified or in-line diluted using PBS at a flow rate ratio of 1:1 or 1:2. mRNA-LNPs containing SARS-CoV-2 spike mRNA were utilized for in vitro applications, and mRNA-LNPs containing FLuc mRNA were used for in vivo biodistribution studies. (b) Western blot results after cell treatment with SARS-CoV-2 spike mRNA-LNPs diluted to the same concentration (spike protein: 190 kDa, GADPH: 37 kDa). (c) Bioluminescence imaging at specific time points following intravenous injection of FLuc mRNA-LNP into male BALB/c mice. (d) Quantification of total luminescence values for each formulation in groups of male BALB/c mice.

The excellent characteristics of mRNA-LNPs manufactured using DVM have already been demonstrated. However, to highlight the superiority of DVM, additional validation is needed to confirm that mRNA-LNPs produced in DVM exhibit quantitative protein expression in vitro or in vivo as well as simple characteristics such as dispersibility compared to the same formulation through a commercial mixer. Referring to FIG. 16, a commercially widely used toroidal mixer was selected as a comparison group for this validation because it can produce mRNA-LNPs with very high characteristics. To perform accurate and diverse evaluations of mRNA-LNP formulations, the inventors of the present invention selected SARS-CoV-Spike-mRNA for in vitro studies and FLuc-mRNA for in vivo biodistribution evaluations (See (a) of FIG. 16).

Toroidal mixers have a much smaller internal volume per structure than DVMs, approximately 23.7 times smaller. (The volume per toroidal mixer internal structure is calculated based on examples disclosed in the patent.) However, to perform an accurate performance comparison analysis, three different formulations were performed using common flow rates (lipid solution 3 mL/min; mRNA solution 9 mL/min) on both the DVM and the toroidal mixer under identical manufacturing conditions, including in-line dilution in PBS. In addition, after formulation, diafiltration was performed for purification along with concentration adjustment. The characteristics of purified LNP samples were compared after protein expression in vitro and in vivo.

The importance of nanoparticle homogeneity was evident from the Western blot results. Each of the identical formulations produced using the toroidal mixer exhibited similar physical and chemical characteristics, and the optimized formulation produced using DVM had a Q value of 0.39, which was approximately 2.4 times higher than the optimal formulation produced using the toroidal mixer (see Table 1).

**Table 1. Comparison of DVM and commercial toroidal mixer in purified mRNA-LNP characteristics under identical formulation conditions. The ratios shown in the "In-line dilution" section represent the flow rate ratios of the entire LNP solution and PBS (pH 7.4).**

| In vitro/In vivo | Mixer | In-line dilution | mRNA | Size(d,nm) | PDI | EE(%) |
|---|---|---|---|---|---|---|
| In vitro | DVM | - | SARS-CoV-Spike | 92 | 0.10 | 91.81 |
| In vitro | DVM | 1:1 | SARS-CoV-Spike | 85 | 0.03 | 94.97 |
| In vitro | DVM | 1:2 | SARS-CoV-Spike | 76 | 0.09 | 92.65 |
| In vitro | Toroidal | - | SARS-CoV-Spike | 95 | 0.09 | 93.7 |
| In vitro | Toroidal | 1:1 | SARS-CoV-Spike | 87 | 0.07 | 94.85 |
| In vitro | Toroidal | 1:2 | SARS-CoV-Spike | 81 | 0.10 | 95.37 |
| In vivo | DVM | - | Firefly luciferase | 83 | 0.07 | 95.66 |
| In vivo | DVM | 1:1 | Firefly luciferase | 87 | 0.06 | 94.98 |
| In vivo | DVM | 1:2 | Firefly luciferase | 88 | 0.05 | 96.49 |
| In vivo | Toroidal | - | Firefly luciferase | 83 | 0.08 | 94.18 |
| In vivo | Toroidal | 1:1 | Firefly luciferase | 82 | 0.07 | 94.57 |
| In vivo | Toroidal | 1:2 | Firefly luciferase | 79 | 0.07 | 95.55 |

Similar protein expression results were obtained for formulations with similar physicochemical characteristics. In contrast, the optimized process using DVM, which showed a 2-fold or more difference in polydispersity index (PDI) despite similar size and encapsulation efficiency, showed approximately 1.8-fold higher protein expression after quantification (FIG. 16(b)).

For comparative studies on the in vivo mRNA delivery effects of mRNA-LNP formulations prepared using different micromixers, the same manufacturing conditions were maintained while only the mRNA component was changed during formulation. Similarly, no significant differences in characteristics were observed between formulations prepared using DVM and toroidal mixers for in vivo applications. When comparing luminescence intensity over time after intramuscular (IM) injection, it shows similar performance among groups using the same manufacturing process, indicating similar results. After 6 hours, the highest level of luminescence with similar intensity was observed in all groups. This luminescence phase lasted for 72 hours and ultimately resulted in complete removal after 144 hours ((c) and (d) of FIG. 16).

The results of bioluminescence over time demonstrate similar biological responses and consistency in mRNA delivery between LNPs produced by the DVM and toroidal mixer. In essence, the study results of the inventors of the present invention demonstrate that despite the larger capacity, the DVM platform exhibits precise processing capabilities with robust system control, resulting in mRNA-LNP characteristics similar to those achieved with commercially available toroidal mixers. In addition, the results of the inventors of the present invention demonstrate similar levels of mRNA delivery efficiency in both in vitro and in vivo settings.

### Continuous operation of DVM

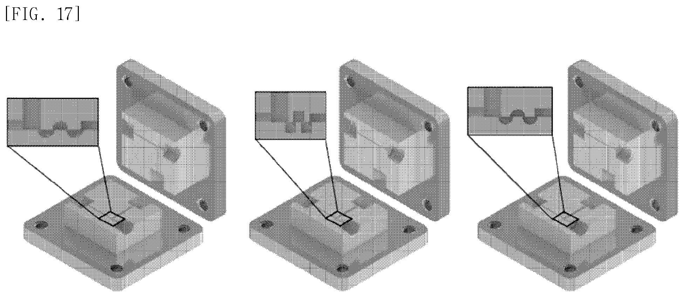

FIG. 17 is a schematic diagram of the assembly type of a 3D-printed microfluidic mixing structure.

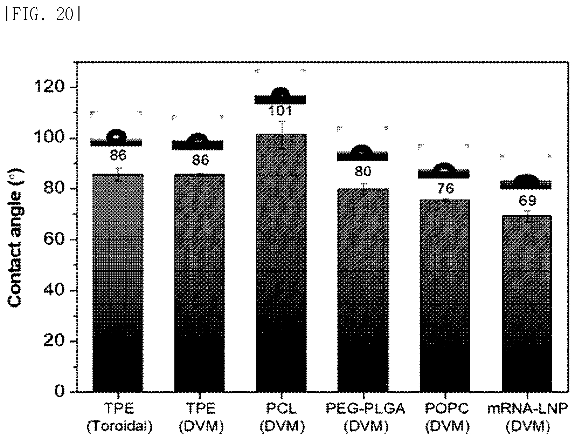

In the DDS industry, maintaining consistent nanoparticle characteristics is critical to continuous manufacturing. A major factor affecting the performance of the reactor is the agglomerates that form inside the mixer during operation, which are the starting point for clogging. The inventors of the present invention configured the previously compared SVM and NVM apparatuses into an assembly-type DVM, as shown in FIG. 20, so that the inside of the apparatus can be viewed to explain the performance of the DVM continuity.

FIG. 18 is a graph of continuous production of TPE nanoparticles for long-term use validation. (a) Standardized size distribution of initial TPE nanoparticles with identical flow rate conditions generated by different microfluidic mixing structures. (b) Real-time analysis of the pressure difference inside the microfluidic mixing structure and (c) the average size of the nanoparticles. (d) Fluorescence visualization of clogged TPE nanoparticles under the same UV irradiation intensity within the decomposed structure. (e) Quantified ratio between the weight of clogged aggregate and the volume of a single structure.

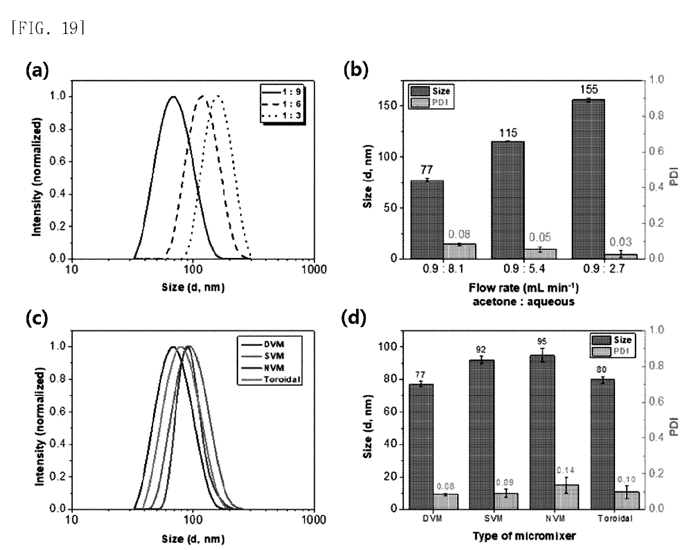

FIG. 19 shows (a) normalized size distribution and (b) PDI values and numerical Z-avg sizes of TPE nanoparticles via DVM at various flow rate ratios (FRR = 3, 6, 9); and (c) normalized size distribution and (d) numerical Z-avg sizes and PDI values under fixed flow rate conditions of the microfluidic mixing structure (acetone 0.9 mL/min, DI water 8.1 mL/min).

Referring to FIGS. 18 and 19, a commercial reactor, a transparent toroidal mixer, and a total of four reactors were again compared to confirm the clogging phenomenon during continuous nanoparticle production. For typical visualization images, FITC-based fluorescent molecules are chosen because of their strong luminescence in solution. On the other hand, existing FITC emits fluorescence in its molecular state in solution, so it is not effective for long-term imaging and tracking. Tetraphenylethylene (TPE), one of the aggregation-induced emission (AIE) molecules, was chosen for visualization and quantification of clogging tests because it allows direct observation of nanoparticles that remain trapped inside the channels and do not lose their photoluminescence over long periods of time. When the FRR was increased to 9, DVM produced highly uniform TPE nanoparticles with a minimum size of 77 nm, followed by 80 nm in the toroidal mixer, 92 nm in the slow-mixing SVM, and 95 nm in the NVM (see FIG. 18(a) and FIG. 19).

FIG. 20 is a nanoparticle contact angle between a 3D-printed microfluidic mixing structure according to an exemplary embodiment of the present invention and a toroidal mixer.

Referring to FIG. 20, since the wetting behaviors of TPE nanoparticles and colloidal solution and the material of the mixer may be different, the contact angle of TPE nanoparticles on the surfaces of the toroidal reactor and DVM was measured to confirm equivalent interaction.

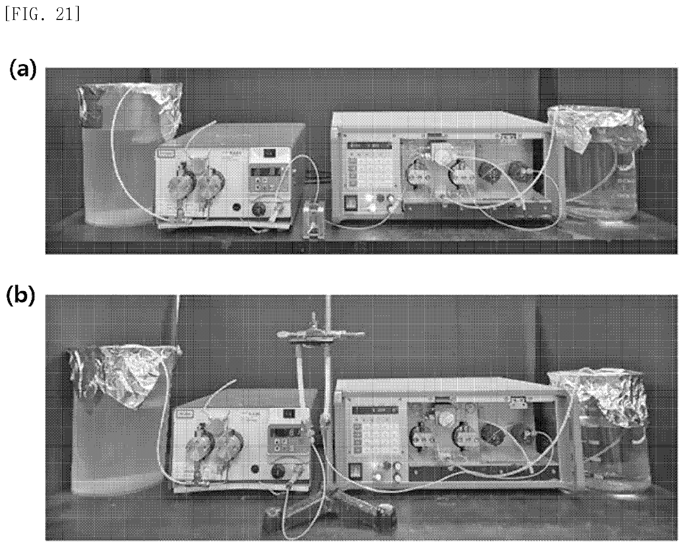

FIG. 21 is a real working image for the continuous production of TPE nanoparticles using a 3D printed (a) microfluidic mixing structure according to an exemplary embodiment of the present invention and (b) a toroidal mixer.

Referring to FIG. 21, the solution was injected using two HPLC pumps with real-time pressure measurement function to monitor the pressure change inside the micromixer based on the upper experimental conditions.

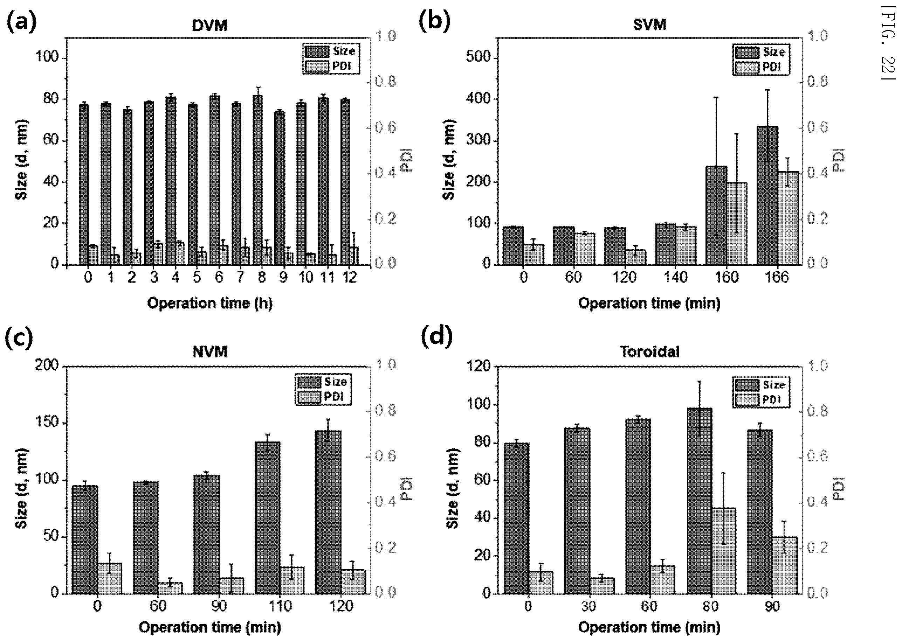

FIG. 22 shows real-time observation values of TPE nanoparticles generated through (a) a microfluidic mixing structure (DVM) according to an exemplary embodiment of the present invention, (b) an SVM, (c) an NVM, and (d) a toroidal mixer.

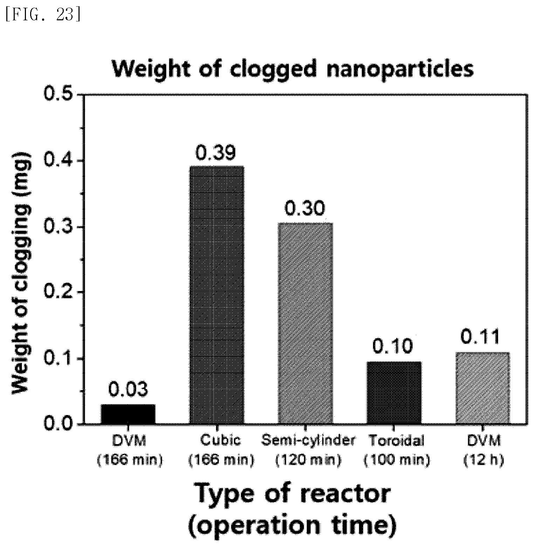

FIG. 23 shows the weight of nanoparticles clogged inside the microfluidic mixing structure, quantified using UV-vis.

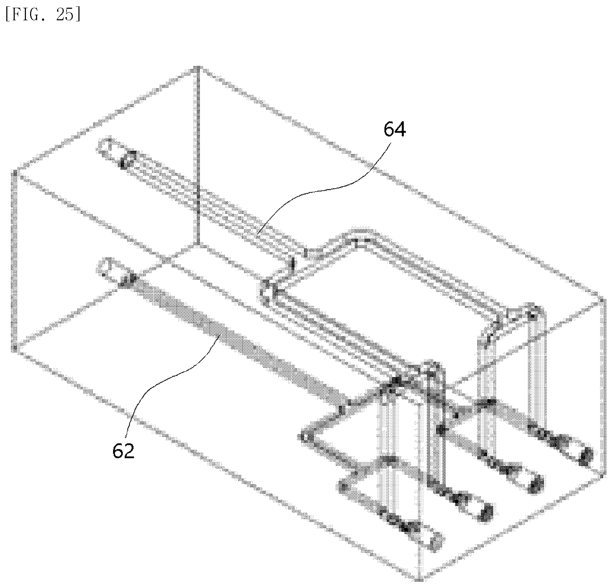

Referring to FIG. 22, continuous DLS analysis was also performed to confirm the change in nanoparticle characteristics in real time. TPE nanoparticles with consistent physical properties were produced in all mixers over a period of approximately 1 hour. However, the pressure difference between the SVM and NVM and the toroidal mixer was found to increase rapidly to 250 psi or more, dramatically changing particle characteristics (FIG. 18(b) to (c), FIG. 25).

Due to this high pressure, leakage began to occur at 166 minutes and 120 minutes in the assembly-type SVM and NVM reactors, respectively. However, the pressure difference in the DVM was limited to 15 psi (460 min later), and despite continuous pump operation for a total of 12 hours, the pressure inside the mixer remained low again close to the initial level. In addition, after 12 hours of continuous operation, the PDI consistently displayed values below 0.1 while the particle size varied up to 5 nm. To visualize and measure the relative clogging to the DVM, the DVM was once again activated using a new reactor at 166 min, the maximum running time of the rest of the reactor.

After continuous operation (until leakage from the SVM and NVM, 166 min and 12 hr) the micro mixer was redissolved with deionized water flowing for 1 min at a slow flow rate to remove the remaining solution without any effect on the clogged aggregate. The degree of clogging of the micro mixer was examined using a microscope (FIG. 18 (d)).

For accurate visualization, the fluorescence of nanoparticles inside the baffle was observed under UV irradiation of the same intensity. For the SVM, NVM and toroidal mixers, a much greater luminescence intensity was observed, especially when the pressure was increased due to obstacles inside the reactor. In contrast, the DVM, which operated for 166 minutes, showed almost no clogging, which could be considered as being in its initial state. The DVM run for 12 hours was similar in that it coated the nanoparticles on the inner reactor walls of the baffle, except for the angular parts such as the inlet and outlet sections. Acetone was used to dissolve the remaining nanoparticle aggregates inside the mixer, and each amount was measured by UV-vis at 330 nm. Considering the volume of a single structure, a similarly operated DVM was found to have a clogging reduction ratio that was more than 10 times lower than other types of baffles and a clogging ratio that was about 62 times lower than a toroidal mixer (see FIG. 18(e) and FIG. 23).

This demonstrates that the relatively large reactor volume enabled by 3D printing can reduce the volume of agglomerated nanoparticles compared to toroidal mixers. In addition, based on the simulation results shown above, the formation of aggregates is prevented due to strong vortex strength. Overall, this validates that DVM is capable of sustainably producing nanoparticles at a selective scale.

### Description of a microfluidic mixing apparatus for mass production

FIG. 24 is a conceptual diagram of an integrated mass production apparatus by parallelization of a flow distributor and a 3D microfluidic mixing structure for nanoparticle manufacturing. (a), (b), and (c) a conceptual diagram of a 3D printing integrated microfluidic mixing structure (4/8/16N-DVM) equipped with a uniform flow distributor and a photograph of the manufactured apparatus. (d), (e) After supplying rhodamine B staining solution and methylene blue staining solution, UV spectrum (665 nm) analysis of the solutions collected at each outlet, total flow rate of 36 mL/min., maldistribution factor (MF) according to the total flow rate of the 4N-DVM apparatus (ethanol flow rate: water flow rate = 1:9).

FIG. 25 is a block diagram in which 4 microfluidic mixing structures are integrally formed in parallel according to an exemplary embodiment of the present invention.

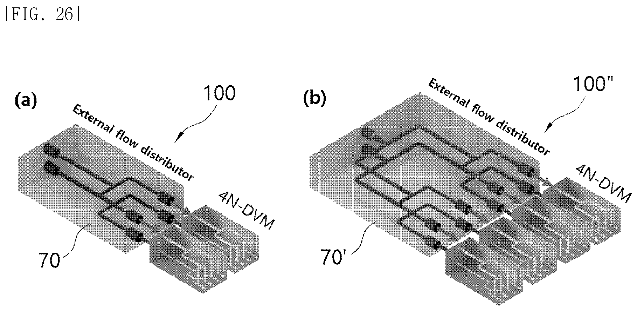

FIG. 26 is a conceptual diagram of an assembly-type mass production mixing apparatus of an external flow distributor and a plurality of 4N-DVMs. (a) a conceptual diagram of a 3-piece-assembled 8N-DVM, (b) a conceptual diagram of a 5-piece-assembled 16N-DVM.

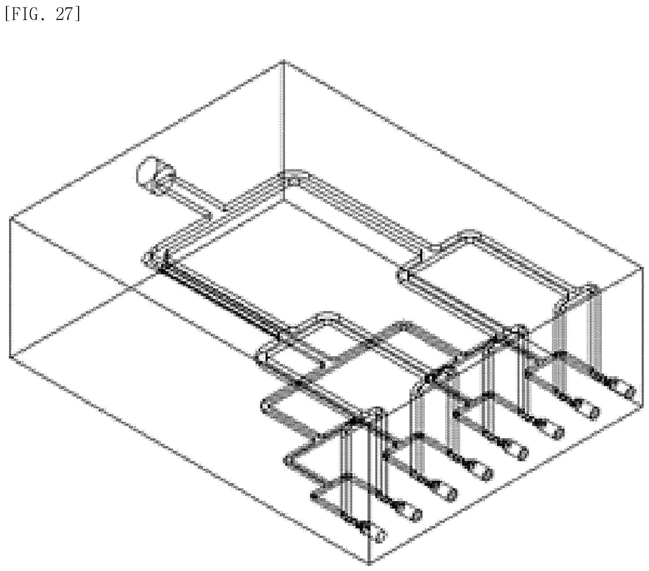

Recently, as the therapeutic capabilities of lipid nanoparticles and polymer nanoparticles have been demonstrated, mass production of nanoparticles for medical purposes has been demanded. Accordingly, the inventors of the present invention designed a mixing apparatus for mass production of nanoparticles by arranging optimized 3D baffle single structures for nanoparticle production in parallel and integrated as shown in FIGS. 24 and 25 and integrating a flow distributor that evenly supplies good solvent and poor solvent to the apparatus. The integrated flow distribution device manufactured using a 3D printer is directly connected to the mixer, which has the great advantage of reducing flow distribution deviation. The flow distribution device was manufactured to have a length of 0.05 Re · D (Re: Reynolds number, D: hydraulic diameter) (ACS Sustainable. Chem. Eng, 2018, 6, 422-429) and a width of 1/√ 2 for each branch (ACS Central Science, 8, 1, 43-50, 2022) to stabilize the flow rate within each branch pipe and prevent the uneven phenomenon of the fluid flowing to one side due to inertia (FIGS 27 (a), (b), and (c)). The detailed dimensions of the finally manufactured integrated reactors for mass production were calculated as shown in Tables 2 and 3.

**Table 2. Detailed dimensions of each section of the poor solvent channel of the integrated 4/8/16N-DVM for mass production (in mm)**

| Nth distribution section | 4N-DVM | | | 8N-DVM | | | 16N-DVM | | |
|---|---|---|---|---|---|---|---|---|---|
| | width | height | length | width | height | length | width | height | length |
| 1 | 0.9 | 0.9 | 23.0 | 0.9 | 0.9 | 11.8 | 0.9 | 0.9 | 6.80 |
| 2 | 1.3 | 0.9 | 18.5 | 1.3 | 0.9 | 25.3 | 1.3 | 0.9 | 6.00 |
| 3 | 1.8 | 0.9 | 13.5 | 1.8 | 0.9 | 19.4 | 1.8 | 0.9 | 25.3 |
| 4 | | | | 2.6 | 0.9 | 14.4 | 2.6 | 0.9 | 19.4 |
| 5 | | | | | | | 3.6 | 0.9 | 11.4 |

**Table 3. Detailed dimensions of each section of the good solvent channel of the integrated 4/8/16N-DVM for mass production (in mm)**

| Nth distribution section | 4N-DVM | | | 8N-DVM | | | 16N-DVM | | |
|---|---|---|---|---|---|---|---|---|---|
| | width | Height | length | width | height | length | width | height | length |
| 1 | 0.5 | 0.5 | 28.7 | 0.5 | 0.5 | 31.4 | 0.5 | 0.5 | 12.2 |
| 2 | 0.7 | 0.5 | 8.00 | 0.7 | 0.5 | 10.7 | 0.7 | 0.5 | 14.2 |
| 3 | 1.0 | 0.5 | 5.50 | 1.0 | 0.5 | 8.00 | 1.0 | 0.5 | 10.7 |
| 4 | | | | 1.4 | 0.5 | 6.40 | 1.4 | 0.5 | 8.00 |
| 5 | | | | | | | 2.0 | 0.5 | 6.40 |

First, by applying the above conditions, an integrated mixing apparatus for mass production, in which four single units were parallelized, was fabricated, and the distribution deviation factor (MF) values per mixer were calculated according to the optimized flow rate and total flow rate for the production of lipid nanoparticles. To accurately quantify the distribution of the two solutions at each outlet, rhodamine B dye was mixed in ethanol and methylene blue dye was mixed in water, and the absorption intensity at 665 nm of the solutions from each outlet was measured using UV-vis, and then the MF value was measured. When the wavelength absorption intensity at each outlet was measured at a flow rate of 9 mL/min, which is optimized for the production of lipid nanoparticles, an even flow rate distribution (MF: 1.55%) was confirmed for each outlet. In addition, it was confirmed that even flow distribution was achieved despite the increase in total flow rate.

The previously manufactured integrated mixing apparatus for mass production has the advantage of being easy to manufacture and having high distribution efficiency because there are no errors that occur during the assembly process. However, there is a disadvantage in that partial replacement of components is not possible if problems occur in some channels during use. Accordingly, the inventors of the present invention separately manufactured a 4N-DVM baffle mixing apparatus and a uniform flow distributor 70, 70' having a simple structure complementary to each other and allowing partial replacement of components, and additionally manufactured a mixing apparatus 100' , 100" for mass production in an assembled form (FIG. 26 (a), (b)). The detailed dimensions of the finally manufactured external flow distributor were calculated as shown in Table 3.

**Table 4. Detailed dimensions of each section of the external flow distributor channel of the assembly-type 8/16N-DVM for mass production (in mm)**

| Nth distribution section | 8N-DVM | | | 16N-DVM | | |
|---|---|---|---|---|---|---|
| | width | Height | length | width | height | length |
| 1 | 1.5 | 1.5 | 27.0 | 1.5 | 1.5 | 27.0 |
| 2 | 2.1 | 1.5 | 51.5 | 2.1 | 1.5 | 51.5 |
| 3 | | | | 3.0 | 1.5 | 12.5 |

A nanoparticle production apparatus, in which hemispherical structures are embedded at the ends of each of the sixteen uniform flow distributors supplying good solvent and poor solvent, is connected (eight each on the upper and lower layers) and integrally manufactured using a 3D printer. The integrated mass production mixer enables production at a flow rate of 17.2 L/h while maintaining the physical properties of the nanoparticles (size, particle uniformity, surface charge, and drug encapsulation efficiency). As a result, the integrated manufacturing method of single units (1x) and mass-producing apparatuses (4x, 8x, 16x) using 3D printers shows the possibility of customized manufacturing methods for small/large-scale production needs.

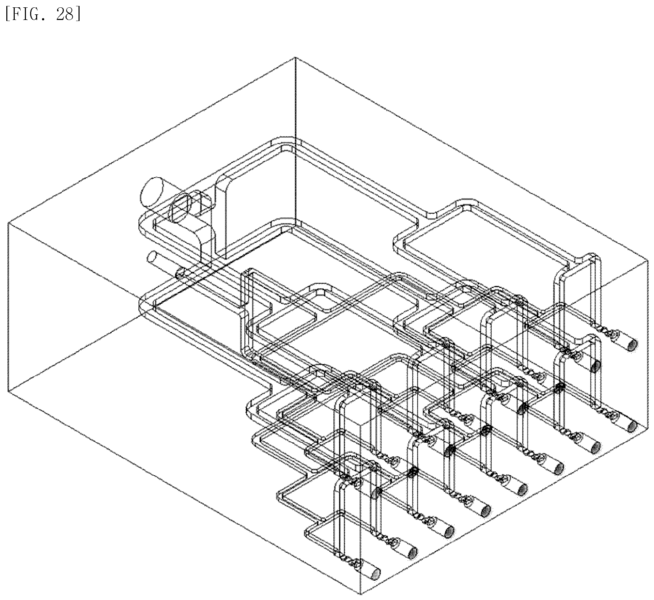

FIG. 27 is a block diagram in which 8 microfluidic mixing structures are integrally formed in parallel according to an exemplary embodiment of the present invention, and FIG. 28 is a block diagram in which 16 microfluidic mixing structures are integrally formed in parallel according to an exemplary embodiment of the present invention.

The microfluidic mixing structure may be configured as in FIG. 26, but may also be manufactured in a form in which multiple mixers are provided in one module as in FIGS. 27 and 28.

### Materials and methods for fabricating and validating performance of 3D hemispherical structures

### Materials

Ethyl alcohol (EtOH), tetrahydrofuran (THF), acetone, sodium chloride, sodium acetate solution, acetonitrile (ACN), 1,1,2,2-tetraphenylethylene (TPE), cholesterol (Chol), and Triton X-100 were purchased from Sigma-Aldrich. 1-palmitoyl-2-oleoyl-sn-glycero-3-phosphocholine (POPC), 1,2-distearoyl-sn-glycero-3-phosphocholine (DSPC), and 1,2-dimyristoyl-rac-glycero-3-methoxypolyethylene glycol-2000 (DMG-PEG 2000) were purchased from Avanti Polar Lipids, Inc. (Heptadecan-9-yl 8-((2-hydroxyethyl)(6-ox0-6-(undecyloxy)hexyl)amino)octanoate)(SM-102) was purchased from XIAMEN SINOPEG BIOTECH CO., LTD. Poly(ethylene glycol) methyl ether block-poly(lactide-co-glycolide) (PEG5k-PLGA55k) was purchased from Creative PEGWorks. SARS-CoV-Spike mRNA and firefly luciferase mRNA (FLuc mRNA) were produced by ST Pharm Co., Ltd.

### Fabrication of 3D printing apparatus

The 3D printing apparatus was designed using computer-aided design (CAD) and software (Autodesk Inventor). To facilitate high-pressure injection of two different solutions, 5-mm threads (1/16" , 1/4-28 Flat-Bottom) were introduced at the injection site at a spacing greater than 10mm, the theoretical flow stabilization length. This modification allowed for a seamless connection to the XP-235 fitting (IDEX Health & Science). The CAD files were then printed using a digital light processing (DLP)-based 3D printer (Pico 2 HD, Asiga) using commercial resin (PlasCLEAR). Each layer was printed at 25 µm and laminated through photocuring. Isopropanol (IPA) was used to remove any uncured residual resin inside the printed apparatus, and then air was flushed through a mixer to further remove the IPA. This process was repeated until no resin was observed at each inlet and outlet. Finally, the cleaned apparatus was post-cured using a UV chamber (Asiga Flash), which transformed the resin into a fully polymerized object.

### Computational fluid dynamics (CFD) simulation of a micromixer

A numerical computational fluid dynamics (CFD) study was performed to evaluate the degree of mixing, turbulent kinetic energy (TKE), and dead volume inside the baffle. The fluid flow inside the micromixer can be described by the Navier-Stokes equation, the volume of fluid (VOF) equation for multiphase fluid, and the mass conservation equation. By assuming steady state, the governing equation of fluid flow can be simplified as follows.
ρv · ∇ v = - ∇ p + µ ∇ ² v + ρg: Navier-Stokes equation
∇ · v = 0: mass conservation equation

Where ρ is the fluid density, v is the fluid linear velocity, p is the pressure, µ is the fluid dynamic viscosity, and g is the gravitational acceleration. The governing equation was solved using appropriate boundary conditions (non-slip boundary conditions at the channel walls, mass flow rates at the inlet, outflow conditions at the outlet, and zero normal gradients for all flow variables except pressure). The equations were discretized based on the finite volume method, and commercial numerical software FLUENT 2022 R2 (ANSYS, INC.) was used for numerical analysis. The calculations used the SST (shear stress transfer) k-Ω standard turbulence model, which combines the k-ε and k-Ω turbulence models. The fluid properties used were the density and viscosity of ethyl alcohol (790 kg m ³, 0.0012 kg m⁻¹ s⁻¹) and water (998.2 kg m³, 0.001003 kg m⁻¹ s⁻¹). The portion of the computational domain where the velocity magnitude falls below 5% of the maximum velocity at a given time is denoted as dead volume.

### Preparation of PCL nanoparticles

PCL was dissolved in THF at a concentration of 1 mg/mL and deionized water was used to precipitate PCL nanoparticles. PCL precursor solution and deionized water were injected using two 20 mL syringes (Air-Tite Products, Inc.). Each syringe was attached to a separate syringe pump (PHD Ultra, Harvard Apparatus), and the flow rate of each pump was controlled using an FRR range of 3 to 9. Each syringe was connected to the poor solvent and polymer inlets, respectively.

### Preparation of PEG-PLGA nanoparticles

PEG 5k-PLGA 55k was dissolved in ACN at a high concentration of 50 mg/ml. Deionized water was used as an antisolvent for precipitation of PEG-PLGA nanoparticles, and the same method as described above for injection was followed.

### Preparation of POPC liposomes

POPC liposomes were prepared by mixing an aqueous solution and a lipid solution. POPC and cholesterol were dissolved in ethanol at a molar ratio of 55:45 to a total concentration of 10mg · mL. Lipid solution and aqueous buffer (saline, 154 mM NaCl) were injected into the lipid inlet and antisolvent inlet, respectively. Self-assembled POPC liposomes were collected from the outlet stream resulting from mixing of two adjacent streams and dialyzed through a dialysis bag (MWCO 12,000 Da, Sigma-Aldrich) against 1,000-fold volume of buffer (10 mM PBS, pH = 7.4) overnight to remove ethanol residue.

### Preparation of mRNA-LNPs for Characterization

After formulation, it was prepared to achieve a final mRNA concentration of 0.10 mg/mL with an ionizable lipid/mRNA mass ratio of 11.5. Briefly, SM-102/DSPC/Chol/DMG-PEG 2000 was dissolved in ethanol at a ratio of 50/10/38.5/1.5 to produce a lipid solution with a total concentration of 20 mg/mL. A 3 M sodium acetate solution was diluted to a 25 mM concentration with nuclease-free water (NFW). The pH of the sodium acetate buffer was adjusted to 5.5 using 100 mM acetic acid solution. Before injection, the lipid solution and sodium acetate buffer were passed through a 0.22 µm syringe filter. FLuc mRNA was then dissolved in sodium acetate buffer. After formulation, mRNA-LNPs were purified using the same purification methods used for liposomes.

### mRNA-LNP formulations for comparison with commercial apparatuses and manufacturing performance

Each mRNA-LNP formulation reached a final mRNA concentration of 0.04 mg/ml with a lipid combination of the same molar ratio. Comparative commercial apparatuses included a toroidal mixer (NanoAssemblr Ignite, Precision NanoSystems). To achieve a complete buffer environment, a centrifugal filter device (Amicon ^{®} Ultra-15) was used for diafiltration. Briefly, the sample to be purified was placed inside the device, the remaining space was filled with a buffer solution, and centrifugation was repeated at 3,500 rpm for 40 minutes until the pH of the solution outside the membrane reached 7.4. For in vitro use, LNPs loaded with SARS-CoV-Spike-mRNA were diluted to achieve an mRNA concentration of 0.02 mg/ml. For in vivo application, LNPs loaded with FLuc-mRNA underwent the same purification process, and the final concentration of mRNA was set to 0.04 mg/ml.

### Cell culture for in vitro studies

HEK 293T cells (ATCC, American Type Culture Collection) were cultured at a density of 1 × 10⁶ cells per well for Western blot analysis. Samples containing SARS-CoV-2 Spike-mRNA, including DVM-LNP and Toroidal-LNP, were dispersed in Opti-MEM (Reduced-Serum Medium, GibcoTM) and treated with 3 µg of mRNA per well. Spike protein expression was measured 24 hours later. After transfection, each well was treated with sterile PBS and 200 µL of 1x lysis buffer (Cell Lysis Buffer, Invitrogen^{™}). Samples were then centrifuged at 13,000 rpm for 10 minutes before being collected for analysis.

### Western blot

Cell extracts were separated by 4-15% gradient SDS-PAGE (4-15% Mini-PROTEAN TGXTM Precast Protein Gels, Bio-Rad). Cell lysate proteins were transferred from the gel to a 0.2 µm PVDF membrane (Mini-format, Bio-Rad) and immunoblotted with recombinant 2019-nCoV spike antibody (SARS-COV-2 Spike RBD Polyclonal Antibody, Elabscience) and GAPDH antibody (sc-47724, Santa Cruz Biotechnology). Analysis was performed with a Western blot imaging system (ChemiDoc MP Imaging System, Bio-Rad) using Western ECL solution (ClarityTM Western ECL Substrate, Bio-Rad).

### In vivo expression of luciferase protein

Male BALB/c mice weighing 22-25 g were randomly divided into seven groups, with n=3 per group. These groups included a negative control group that received PBS, three groups that received DVM-LNP samples containing FLuc-mRNA, and three groups that received Toroidal-LNP samples. Each group was administered 50 µL (containing 5 µg of mRNA) into the right quadriceps femoris muscle of mice. Body weight and bioluminescence of each mouse were measured at seven time points: before administration, 0.5 h, 6 h, 24 h, 48 h, 72 h, and 6 days after administration. Before image acquisition, each mouse was injected with 100 µL of luciferin (D-Luciferin, GoldBio) at a concentration of 15 mg mL⁻¹ into both abdominal cavities, and then images were captured at specified time points using IVIS Spectrum (PerkinElmer, USA). After acquiring images for up to 6 days, a region of interest (ROI) was defined around the injection site, and total flux values were calculated.

### Characterization of nanoparticle size and encapsulation efficiency

DLS analysis was performed using a Zetasizer Nano ZS instrument (Malvern Instruments, Ltd.) to evaluate the size distribution of the synthesized nanoparticles according to their strength. All measurements without liposomes were performed using polymer or lipid solutions at concentrations less than 10% to eliminate potential influence of solvent on the observed particle size changes. For liposomes, DLS analysis was performed prior to the dialysis process due to the high instability of the nanoparticles. For mRNA-LNPs, the encapsulation efficiency of mRNA was characterized using the Quant-iT^{™} RiboGreen assay (ThermoFisher Scientific). Briefly, LNPs were first diluted using TE (Tris EDTA) buffer to a concentration in the range of 4-7 ng µL. Diluted LNPs treated with TE buffer to maintain the LNP structure or 1% v/v Triton X-100 to disrupt the structure and release mRNA were diluted 100-fold and reacted with an equal volume of RiboGreen solution. Free and total mRNA in the formulations were determined using a microplate reader (Hidex Sense, Hidex Oy) with 500 nm excitation and 520 nm emission.

### Long-term operation test of micro mixer

TPE at a concentration of 1 mg/mL was dissolved in acetone. Deionized water was used as an antisolvent for continuous nanoparticle production. The beaker was sealed using parafilm and foil to prevent solvent evaporation. A single HPLC pump (PR-Class, Teledyne SSI) was connected to a deionized water beaker to enable injection at high flow rates. Another HPLC pump (LC-10AD, Shimadzu) was connected to the TPE solution. The outlet of the HPLC pump was connected to 1/4" PTFE tubing through a 316 stainless steel reducer (SS-400-6-1BT, Swagelok) to connect to the toroidal mixer. The end of each tubing was further connected to a 3 mm Stainless Needle Luer-lock tubing connector (KT503s, Needle store) to fit into the inlet of the toroidal mixer. The HPLC pumps were set to operate at a TPE solution flow rate of 0.9 mL/min and a deionized water flow rate of 8.1 mL/min. The pressure of the HPLC pump was continuously monitored in real time during operation to calculate the pressure difference, and the nanoparticles generated at the outlet were collected into different vials for further DLS analysis.

### Visualization and quantification of clogging inside a micromixer

Deionized water was injected at a flow rate of 0.1 mL/min for 1 minute to wash away the remaining solution without affecting the clogged nanoparticles. Afterwards, the micro mixer was dried in an 80°C oven for 30 minutes to remove any water remaining inside. The particles remaining inside were directly visualized and quantified for the toroidal mixer, while for the 3D printed micromixer they were broken down prior to visualization and quantification.

It was placed under UV light (365 nm, approximately 100 mW cm⁻²) and observed under a microscope. The clogged nanoparticles were dissolved using acetone and collected in vials for quantification. The amount of nanoparticles was determined through a calibration curve using TPE in acetone at a wavelength of 330 nm.

Microfluidic systems hold promise in the field of drug delivery due to their ability to produce uniform and size-controlled nanoparticles with high reproducibility and have a wide range of applications in research and clinical settings. However, current micromixers severely lack the capabilities to enable rapid and efficient mixing under minimal flow conditions while preventing clogging and allowing easy scale-up for on-demand production. None of the reported scalable devices have been demonstrated to produce nanoparticles that maintain consistent characteristics for hours of continuous device operation. To address these issues, the inventors of the present invention have developed a mixer that utilizes a three-dimensional structure to maximize the vortex effect within a microfluidic system, thereby enabling high-speed mixing between solutions and efficient dispersion of nanoparticles. The user-friendly DVM platform demonstrates nearly equivalent performance comparable to commercial mixers with much less volume in terms of particle characteristics and protein expression.

Most microfluidic mixers that have been proposed so far and can be easily fabricated using photolithography use flow rates in the range of tens to hundreds of µL/min, which can be challenging in terms of productivity. On the other hand, monolithic hydrodynamic focusing or vortex-focusing mixers require flow rates higher than a few tens of ml/min for rapid mixing, and therefore reagents are consumed considerably during optimization. In addition, the use of high FRR in these mixers may result in a concentration process after nanoparticle generation. The DVM proposed by the inventors of the present invention is a platform that operates at a flow rate of several mL/min and bridges the gap between microfluidic mixers.

The high-speed mixing capability allows the use of relatively large volume micro-mixers in reverse to control high concentrations of precursors and effectively prevent clogging. Integrating antifouling materials with manufacturing components can further reduce adhesion, so research in this area is needed. In addition, the small 3.9 mm nanoparticle production section allows for parallelization of the scale-up process to create a monolithic single mass production module. This approach provides higher flow uniformity distribution compared to using assembly-based external flow distributors. Through the DVM platform, the inventors of the present invention expect to accelerate a wide range of nanoparticle-based medical applications by enabling selective optimization and production of particle characteristics in a monolithic modular unit form.

Although exemplary embodiments of the present invention have been described, the idea of the present invention is not limited to the embodiments set forth herein. Those of ordinary skill in the art who understand the idea of the present invention may easily propose other embodiments through supplement, change, removal, addition, etc. of elements within the same idea, but the embodiments will be also within the idea scope of the present invention.

## Claims

1. A microfluidic mixing structure for mixing a first fluid and a second fluid while moving them in a first direction, the microfluidic mixing structure comprising:
a first mixing unit including a first body which has an inlet through which the first fluid and the second fluid are introduced, and a first space in which the first fluid and the second fluid are mixed; and
a second mixing unit which is provided on the rear side of the first mixing unit when viewed in the first direction, and comprises a second body having a second space through which a mixed fluid obtained in the first mixing unit flows,
wherein the first space in the first mixing unit and the second space in the second mixing unit are provided on opposite sides of an imaginary line extending in the first direction,
wherein the flow direction of the mixed fluid, in which the mixed fluid is introduced from an outlet of the first mixing unit into an inlet of the second mixing unit, is opposite to the first direction, and
the first mixing unit and the second mixing unit comprise curved surfaces that are bilaterally symmetric with respect to the center in the width direction when viewed in the first direction.

2. The microfluidic mixing structure of claim 1, wherein the first space in the first mixing unit and the second space in the second mixing unit comprise one of an elliptical cross-section and a semicircular cross-section when viewed in the first direction.

3. The microfluidic mixing structure of claim 2, wherein the first space in the first mixing unit and the second space in the second mixing unit are formed in a hemispherical shape.

4. The microfluidic mixing structure of claim 1,
wherein the second mixing unit is positioned above the first mixing unit in the up-down direction, and
wherein the inlet of the first mixing unit is arranged to face upward.

5. The microfluidic mixing structure of claim 1, wherein the first space in the first mixing unit and the second space in the second mixing unit are formed with the same area of each inlet and outlet.

6. The microfluidic mixing structure of claim 5, wherein the cross-sectional area of the central portion of the first space in the first mixing unit and the second space in the second mixing unit is formed to be larger than the area of the inlet and outlet of each of the first space and the second space when viewed in the first direction.

7. The microfluidic mixing structure of claim 6, wherein the first space in the first mixing unit and the second space in the second mixing unit have the inlet and outlet of each space formed as a closed curve shape in which a pair of circular arcs of the same diameter face each other and are in contact with each other when viewed in the first direction.

8. The microfluidic mixing structure of claim 1, further comprising a third mixing unit comprising: a third body placed on the rear side of the second mixing unit, when viewed in the first direction, and having a third space through which the mixed fluid passing through the second mixing unit flows.

9. The microfluidic mixing structure of claim 8, wherein the third mixing unit has the same shape as at least one of the first mixing unit and the second mixing unit.

10. The microfluidic mixing structure of claim 9, wherein the third space in the third mixing unit is arranged opposite to the second space in the second mixing unit with respect to an imaginary line extending in the first direction.

11. The microfluidic mixing structure of claim 6, further comprising:
a first fluid inlet pipe through which the first fluid passes so that the first fluid is introduced into the inlet of the first mixing unit; and
a second fluid inlet pipe through which the second fluid passes so that the second fluid is introduced into the inlet of the first mixing unit,
wherein the first fluid inlet pipe and the second fluid inlet pipe are arranged to be orthogonal, and
wherein one of the first fluid inlet pipe and the second fluid inlet pipe is arranged to extend in the first direction.

12. The microfluidic mixing structure of claim 11,
wherein the third space in the third mixing unit has an outlet oriented upward, and
wherein a mixed fluid discharge pipe extending in the first direction is connected to the outlet of the third space.

13. The microfluidic mixing structure of claim 8, wherein the first body, the second body, and the third body are formed as a module integrated into one body.

14. The microfluidic mixing structure of claim 13,
wherein a plurality of first mixing units, a plurality of second mixing units respectively connected to the plurality of first mixing units, and a plurality of third mixing units respectively connected to the plurality of second mixing units are provided inside the module integrated into one body,
wherein a first fluid supply flow passage to which a first fluid supply pipe for supplying the first fluid into the interior of the one body is connected, and a second fluid supply flow passage to which a second fluid supply pipe for supplying the second fluid into the interior of the one body is connected are provided in the one body, and
wherein the first fluid supply flow passage and the second fluid supply flow passage are formed to be branched into a plurality of flow passages to supply the first fluid and the second fluid to the plurality of first mixing units.

15. The microfluidic mixing structure of claim 14,
wherein the module integrated into one body comprises 1, 2, 4, or 8 pairs of mixing flow passages therein, and
wherein each of the plurality of pair of mixing flow passages is formed such that two first mixing units, two second mixing units, and two third mixing units are formed in a pair.

16. A microfluidic mixing apparatus, comprising:
one or more microfluidic mixing structures according to any one of claims 13 to 15;
a first fluid feeder configured to supply the first fluid to each of the one or more microfluidic mixing structures;
a second fluid feeder configured to supply the second fluid to each of the one or more microfluidic mixing structures.

17. The microfluidic mixing apparatus of claim 16, further comprising a flow distributor configured to distribute the first fluid and the second fluid supplied from the first fluid feeder and the second fluid feeder to the one or more microfluidic mixing structures.
